# EUROPEAN PATENT SPECIFICATION

(11) **EP 4 268 816 B1**
(45) Date of publication and mention of the grant of the patent: **25.03.2026**
(21) Application number: 21910857.8
(22) Date of filing: 22.12.2021
(51) Int. Cl.: A61K 31/4184, A61K 9/20, A61K 9/28, A61K 47/14, A61K 47/26, A61K 47/32, A61K 47/36, A61K 47/38, A61P 25/02, A61P 29/00

(54) **ORAL SOLID PREPARATION**
ORALE FESTE ZUBEREITUNG
PRÉPARATION SOLIDE ORALE

(30) Priority: 23.12.2020 JP 2020213086
(43) Date of publication of application: 01.11.2023
(73) Proprietor: Sumitomo Pharma Co., Ltd., Osaka 541-0045 (JP); Alphanavi Pharma Inc., Suita-shi, Osaka 564-0053 (JP)
(72) Inventor: IKARI, Hiroki, Suita-shi, Osaka 564-0053 (JP); KATO, Yuka, Suita-shi, Osaka 564-0053 (JP); MATSUOKA, Makoto, Osaka-shi, Osaka 554-0022 (JP); MATSUI, Yasuhiro, Suita-shi, Osaka 564-0053 (JP); MARUYAMA, Megumi, Suita-shi, Osaka 564-0053 (JP)
(74) Representative: Mewburn Ellis LLP
(86) International application number: PCT/JP2021/047561
(87) International publication number: WO 2022/138717

(56) References cited:
- EP-A1- 2 380 881
- WO-A1-2017/170858
- WO-A1-2019/230937
- WO-A1-2020/017585
- JP-A- 2011 126 915
- JP-A- 2018 184 382
- JP-A- 2019 011 379
- JP-A- 2020 125 358
- JP-A- 2020 169 145
- JP-B2- 5 667 934

## Description

### TECHNICAL FIELD

The present invention relates to an oral solid dosage form comprising N²-{[1-ethyl-6-(4-methylphenoxy)-1H-benzimidazol-2-yl]methyl}-L-alaninamide (hereinafter, also referred to as "the present compound") or a pharmaceutically acceptable salt thereof, or a hydrate or solvate thereof (hereinafter, which are also referred to as "medicament" or "the present medicament", including the present compound) as an active ingredient, which has good dissolution and storage-stability. When the oral solid dosage form is a tablet, the tablet can be prepared while inhibiting tableting troubles from low content to high content of the active ingredient.

### BACKGROUND OF THE INVENTION

It is important to obtain the information of dose and its response in clinical development planning. According to Non-patent literature 1, the purpose of collecting the information of dose-response is to collect necessary information at each stage throughout non-clinical and clinical developments, and utilize that information for subsequent clinical trials and post-marketing use. That is, the purpose is to know the relationship between dose, blood level, and clinical response (i.e., efficacy and safety); find how best to determine an appropriate starting-dose and adjust the dose to meet the needs of a particular patient for a population or individual patient; find a dose at which no further benefit can be expected even by increasing the dose or a dose that is likely to cause intolerable side effects if increased; and take the obtained findings to the next stage. In addition, since the dose may be changed in the middle of clinical development, it is preferable to obtain such information of various oral solid dosage forms that cover a wide range of doses, from low to high doses of the active ingredient.

If the amount of impurities increases during storage, there arises some concerns about adverse effects on the body due to the generation of side effects and loss of marketability due to shortening of the shelf life. In addition, when a solid dosage form is orally administered, it is necessary to make the active ingredient dissolve through the processes of disintegration, dispersion, and dissolution and reach the absorption site in order to exert its effect. Thus, it is desirable to design formulations that can maintain stable and good dissolution to exert and maintain pharmacological effects, since these processes have a great influence on drug absorption.

Patent literature 1 discloses an oral dosage form comprising lurasidone as an active ingredient, pregelatinized starches, a water-soluble excipient, and a water-soluble polymer binder, which has the same dissolution behavior even if the content of the active ingredient varies. However, Patent literature 1 neither discloses nor suggests storage stability, or tableting problems when provided as tablets.

Patent literatures 2 and 4 specifically disclose processes of preparing tablets and powders as production examples with the present compound. However, Patent literature 2 neither discloses nor suggests, as a working example, any dissolution of the present compound as an active ingredient from the oral solid dosage form when the concentration of the present compound is varied, any storage stability of the oral solid dosage form, or further any tableting problems when provided as a tablet. In addition, Patent literature 3 also discloses examples of formulations comprising the present compound, but all of them relate to preparations for external use.

### PRIOR ART

### (Patent Reference)

[Patent literature 1] JP 5285105 B
[Patent literature 2] WO 2010/074193
[Patent literature 3] WO 2020/017585
[Patent literature 4] JP 5667934 B

### (Non-Patent Reference)

[Non-patent literature 1] DOSE-RESPONSE INFORMATION TO SUPPORT DRUG REGISTRATION (ICH E4)

### SUMMARY OF INVENTION

### (Technical Problem)

The purpose of the present invention is to provide an oral solid dosage form which has good dissolution and storage stability, and when the said oral solid dosage form is a tablet, the tablet can be prepared while inhibiting tableting troubles from low content to high content of the active ingredient.

### (Solution to Problem)

The present inventors have studied a formulation composition which can cover a wide range of the contents of the present compound, 5 mg to 100 mg per tablet, and have found that the lower the drug concentration in the formulation became, the more impurities (especially Impurity 1) increased and the storage stability worsened. On the other hand, when the concentration of the present compound in the formulation was increased, tableting problems such as a phenomenon of the compound adhering to the surface of the punch (so-called "sticking") and a phenomenon of the surface peeling off (so-called "capping") in the tableting process were observed, and the higher the drug concentration, the more noticeable the tableting problems, and furthermore the problem that the dissolution rate of the present compound may be delayed also became apparent.

The present inventors have extensively studied to solve the above problems, and then have found that it is possible to provide an oral solid dosage form having good dissolution and storage stability by using pregelatinized starches, or pregelatinized starches and low substituted hydroxypropylcellulose as a disintegrant, and hydroxypropylcellulose as a binder. In addition, regarding the tableting problems caused by high concentration of the present compound in a tablet, it has been found that the use of sodium stearyl fumarate instead of magnesium stearate which is a usual lubricant can be greatly improved. However, instead, the storage stability problem due to the increase of impurities occurred. Surprisingly, however, the present inventors have found that the formulation composition comprising the disintegrant and the binder of the present invention can prevent the tableting troubles from low content to high content of the active ingredient, and can provide excellent storage stability while maintaining good dissolution properties, even when preparing a tablet comprising sodium stearyl fumarate as a lubricant. Based upon the new findings, the present invention has been completed.

The present invention provides the following items which are set out in the appended claims.

(Item 1) An oral solid dosage form comprising
   (i) N²-{[1-ethyl-6-(4-methylphenoxy)-1H-benzimidazol-2-yl]methyl}-L-alaninamide or a pharmaceutically acceptable salt thereof, or a hydrate or solvate thereof,
   (ii) a disintegrant, wherein the disintegrant comprises pregelatinized starches;
   (iii) a binder; and
   (iv) a lubricant, wherein the lubricant comprises sodium stearyl fumarate.
(Item 2) The oral solid dosage form of Item 1, wherein the pregelatinized starches comprise a pregelatinized starch and/or a partly pregelatinized starch.
(Item 3) The oral solid dosage form of Item 2, wherein the pregelatinized starches comprise a partly pregelatinized starch.
(Item 4) The oral solid dosage form of any one of Items 1 to 3, wherein the disintegrant further comprises a cellulose-type disintegrant.
(Item 5) The oral solid dosage form of Item 4, wherein the cellulose-type disintegrant comprises low substituted hydroxypropylcellulose and/or croscarmellose sodium.
(Item 6) The oral solid dosage form of Item 1, wherein the disintegrant further comprises low substituted hydroxypropylcellulose.
(Item 7) The oral solid dosage form of Item 1, wherein the disintegrant further comprises croscarmellose sodium.
(Item 8) The oral solid dosage form of any one of Items 1 to 7, wherein the binder is a water-soluble polymer binder.
(Item 9) The oral solid dosage form of Item 8, wherein the water-soluble polymer binder comprises one or a mixture of two or more selected from the group consisting of hydroxypropylcellulose, hydroxypropylmethylcellulose, and polyvinyl alcohol.
(Item 10) The oral solid dosage form of Item 8, wherein the water-soluble polymer binder comprises hydroxypropylcellulose.
(Item 11) The oral solid dosage form of any one of Items 1 to 10, wherein the content of the disintegrant is 10 - 50 wt % per 100 wt % of the whole dosage form.
(Item 12) The oral solid dosage form of any one of Items 2 to 11, wherein the content of the disintegrant other than the pregelatinized starches is 5 - 30 wt % per 100 wt % of the whole dosage form.
(Item 13) The oral solid dosage form of any one of Items 1 to 12, wherein the content of the pregelatinized starches is 10 - 30 wt % per 100 wt % of the whole dosage form.
(Item 14) The oral solid dosage form of any one of Items 10 to 13, wherein the content of the hydroxypropylcellulose is 1 - 10 wt % per 100 wt % of the whole dosage form.
(Item 15) The oral solid dosage form of any one of Items 1 to 14, wherein the content of the N²-{[1-ethyl-6-(4-methylphenoxy)-1H-benzimidazol-2-yl]methyl}-L-alaninamide or a pharmaceutically acceptable salt thereof, or a hydrate or solvate thereof is 0.5 - 70 wt % per 100 wt % of the whole dosage form.
(Item 16) The oral solid dosage form of any one of Items 1 to 15, wherein the content of the N²-{[1-ethyl-6-(4-methylphenoxy)-1H-benzimidazol-2-yl]methyl}-L-alaninamide or a pharmaceutically acceptable salt thereof, or a hydrate or solvate thereof is 1 - 50 wt % per 100 wt % of the whole dosage form.
(Item 17) The oral solid dosage form of any one of Items 1 to 16, further comprising an excipient.
(Item 18) The oral solid dosage form of Item 17, wherein the excipient comprises a water-soluble excipient.
(Item 19) The oral solid dosage form of Item 18, wherein the water-soluble excipient comprises mannitol.
(Item 20) The oral solid dosage form of any one of Items 1 to 19, wherein the content of the sodium stearyl fumarate is 2 - 10 wt % per 100 wt % of the whole dosage form.
(Item 21) The oral solid dosage form of any one of Items 19 to 20, which is prepared by granulating a mixed powder comprising the N²-{[1-ethyl-6-(4-methylphenoxy)-1H-benzimidazol-2-yl]methyl}-L-alaninamide or a pharmaceutically acceptable salt thereof, or a hydrate or solvate thereof, the disintegrant, and the water-soluble excipient, with a solution of the binder.
(Item 22) The oral solid dosage form of any one of Items 1 to 21, which is film-coated with a coating agent.
(Item 23) The oral solid dosage form of any one of Items 1 to 22, which is for use in treating and/or preventing pain.
(Item 24) The oral solid dosage form for use of Item 23, wherein the pain is peripheral neuropathic pain.

### (Effect of the Invention)

The oral solid dosage form of the present invention comprises pregelatinized starches, or pregelatinized starches and low substituted hydroxypropylcellulose as a disintegrant, and hydroxypropylcellulose as a binder, thereby it is possible to provide an oral solid dosage form having good dissolution and storage stability. Furthermore, the formulation composition in the present invention can prevent the tableting troubles from low content to high content of the present compound, and can provide excellent storage stability while maintaining good dissolution properties, even when preparing a tablet comprising stearyl fumarate as a lubricant which has concerns about adverse effects on storage stability.

### DESCRIPTION OF EMBODIMENTS

Hereinafter, the present invention is explained in more detail. The embodiments shown below are provided for a better understanding of the invention, but the scope of the present invention is not limited thereto. In addition, the following embodiments may be used alone or in combination.

The "mean particle size" used herein means the cumulative 50 % particle size D₅₀ in volume-based measurement of powder particles. The mean particle size is measured on a volumetric basis with a laser diffraction particle size distribution analyzer (for example, Particle Viewer of Powrex corp., SALD-3000J of SHIMADZU CORPORATION, or HELOS & RODOS of Sympatec GmbH).

The "tableting trouble" used herein means a phenomenon of the compound adhering to the surface of the punch (so-called "sticking") and a phenomenon of the surface peeling off of the tablet (so-called "capping") in the tableting.

The "content" used in the present invention means compounding amount or content amount (wt/wt) per 100 wt % of the whole dosage form, unless otherwise indicated. The content of the present medicament is preferably 0.1 - 96 wt %, 0.5 - 70 wt %, 1 - 50 %, or 1 - 40 % per 100 wt % of the whole dosage form.

The "excellent storage stability" used herein means that the increase of impurities is little about the purity evaluation in storage stability test and good dissolution properties are maintained in the dissolution test after storage.

The "storage" denotes to put the prepared dosage form in a suitable container, to store it, or keep it. In storage, the container may be sealed or open, and may or may not be protected from light. The storage includes, for example, ordinary temperature storage, room temperature storage, cold place storage, refrigerated storage, and frozen storage. The temperature for storage includes, but is not limited to, standard temperature: 20°C, ordinary temperature: 15 - 25°C, room temperature: 1 - 30°C, lukewarm temperature: 30 - 40°C, cold place: 1 - 15°C, refrigerate: 2 - 6°C, freeze: about -20°C - -18°C, etc. The relative humidity (% RH) for storage may be any one of 0 - 100 % RH, preferably 40 - 60 %RH, but should not be limited thereto. The storage period includes, but is not limited to, hours, days, weeks, months, or years. The parameters in storage test can be appropriately selected according to the properties and storage conditions of the dosage form. The "storage stability test" is a stability test to confirm whether the quality of the prepared dosage form is maintained, which uses storage conditions that promote chemical or physical changes in the dosage form. The test results can be used to assess the chemical effects of long-term storage under prescribed storage conditions. It can be also used to assess the impact of short-term deviations from storage methods that may occur during transport. Depending on the difference in temperature/relative humidity, there are long-term tests (eg 25°C/60 % RH), accelerated tests (eg 40°C/75 % RH), stress tests (eg 50°C/85 % RH), etc. The accelerated test or stress test in the present invention is performed by setting the conditions of a thermo-hygrostat to higher temperature/higher relative humidity (e.g., 40°C/75 % RH or 50°C/85 % RH), and putting the prepared solid dosage form in an appropriate container (e.g., HDPE Bottle (material: high density polyethylene (HDPE), or amber screw-cap test tube (material: glass)), and storing it under unsealed or closed in the thermo-hygrostat for a certain period (e.g., each 1, 3, 6 months, or 2 weeks, 4 weeks). It is necessary to keep the amount of impurities within the specified amount in the storage stability test in order to provide excellent-quality dosage forms to patients. As a specified amount of impurities, 0.2 % or less per one impurity is a guideline.

The "good dissolution properties" used herein means, for example, the dissolution rate is preferably 80 % or more, more preferably 85 % or more at 15 minutes when the dissolution test of the dosage form is performed under the following dissolution test conditions according to the Japanese Pharmacopoeia, 16th Edition, Dissolution Test, Apparatus 2 method.
Dissolution medium: buffer adjusted to pH 4.5 using 0.05 mol/L disodium hydrogen phosphate and 0.025 mol/L citric acid
Paddle Rotating speed: 50 rpm
Volume of dissolution medium: 900 mL
Temperature of dissolution medium: 37 ± 0.5°C

### [1] Oral solid dosage form

The "oral solid dosage form" denotes a solid dosage form having a certain shape that is orally administered. The solid dosage form includes those formulated into dosage forms such as tablets, capsules, granules, fine granules, pills, and powders.

The oral solid dosage form disclosed herein particularly intend to those formulated into tablets, capsules, granules, and fine granules. A preferred example of the oral solid dosage form is tablets or capsules. A more preferred example of the oral solid dosage form is tablets.

The oral solid dosage form disclosed herein (for example, tablets) may be film-coated with a coating agent to facilitate administration or prevent decomposition of the active ingredient. Thus, the oral solid dosage form disclosed herein may be film-coated tablets. Film-coated tablets are generally prepared by film-coating uncoated tablets with a suitable coating agent such as polymer compounds.

The oral solid dosage form disclosed herein comprises (i) medicament, (ii) a disintegrant, (iii) a binder, and (iv) a lubricant, and may optionally comprise (v) an excipient, and further may optionally comprise other (vi) additives to the extent that they do not affect the formulation function of the present invention.

The tablets of the present invention include uncoated tablets, film-coated tablets which are prepared by film-coating the surface of uncoated tablets (herein which may be also referred to as FC tablet), and sugar-coated tablets which are prepared by sugarcoating the surface of uncoated tablets; and preferably uncoated tablets and film-coated tablets.

### (i) Medicament

The "medicament" disclosed herein denotes N²-{[1-ethyl-6-(4-methylphenoxy)-1H-benzimidazol-2-yl]methyl}-L-alaninamide (the present compound) or a pharmaceutically acceptable salt thereof, or a hydrate or solvate thereof, which is the active ingredient of the present invention.

The "pharmaceutically acceptable salt" disclosed herein includes hydrochloride, citrate, fumarate, maleate, phosphate, succinate, sulfate, tartrate, besylate, and hydrobromide.

The "hydrate" in the present invention means a solid state in which water molecules are contained in the molecule of the present compound at a certain ratio, and the "solvate" refers to the case where the water molecules are replaced by organic solvent molecules. The organic solvent capable of forming a solvate in the present invention can be appropriately selected depending on the type and property of the substance, and includes, for example, alcohols such as ethanol and propanol, and general solvents such as acetone and ethyl acetate. The number of solvent molecules in the hydrate and solvate can be appropriately selected depending on the stability of the solvate and the like.

The content of the present medicament is generally 0.1 - 96 wt % per 100 wt % of the whole dosage form, which includes, for example, preferably 0.5 - 70 wt %, more preferably 1 - 50 wt %, and even more preferably 1 - 40 wt %.

The medicament may be milled to a desired particle size, as appropriate. The mean particle size of the medicament includes, for example, generally 0.1 - 100 µm, preferably 0.1 - 80 µm, more preferably 0.1 - 50 µm, and even more preferably 1 - 10 µm. The mean particle size of the medicament may be within the above range as the starting material, and may vary during the manufacturing process or the like.

### (ii) Disintegrant

"Disintegrant" denotes an ingredient to be added for the purpose of disintegrating/dispersing solid dosage form such as tablets and granules into particles.

The "disintegrant" includes, for example, cornstarch, pregelatinized starches, sodium carboxymethyl starch, croscarmellose sodium, microcrystalline cellulose, low substituted hydroxypropylcellulose, carmellose, carmellose calcium, carboxymethylethylcellulose, and crospovidone; preferably pregelatinized starches, croscarmellose sodium, low substituted hydroxypropylcellulose, carmellose, and carmellose calcium; more preferably pregelatinized starches, croscarmellose sodium, and low substituted hydroxypropylcellulose; and even more preferably pregelatinized starches and low substituted hydroxypropylcellulose. The solid dosage form disclosed herein comprises pregelatinized starches. The disintegrant may additionally be one selected from those mentioned above or a combination of two or more thereof.

Another preferred embodiment of the "disintegrant" includes a combination of pregelatinized starches and a cellulose-type disintegrant. The use of pregelatinized starches alone or a combination of pregelatinized starches and a cellulose-based disintegrant as a disintegrant can suppress the decrease in the dissolution rate caused by changing the medicament content. In addition, the use of such disintegrant may bring in a dosage form having a good storage stability even for low-content preparations which may have a concern about increased impurities.

The "cellulose-type disintegrant" includes, for example, croscarmellose sodium, microcrystalline cellulose, low substituted hydroxypropylcellulose, carmellose, carmellose calcium, and carboxymethylethylcellulose among the above-exemplified disintegrants; preferably croscarmellose sodium, microcrystalline cellulose, low substituted hydroxypropylcellulose, and carmellose; more preferably low substituted hydroxypropylcellulose and croscarmellose sodium; and even more preferably low substituted hydroxypropylcellulose.

The content of the disintegrant is generally 0.1 - 80 wt % per 100 wt % of the whole dosage form, which includes, for example, preferably 1 - 70 wt %, more preferably 10 - 50 wt %, and even more preferably 15 - 30 wt %.

The content of the disintegrant other than pregelatinized starches (for example, a cellulose-type disintegrant such as low substituted hydroxypropylcellulose) is generally 1 - 50 wt % per 100 wt % of the whole dosage form, which includes, for example, preferably 5 - 40 wt %, more preferably 5 - 30 wt %, and even more preferably 10 - 20 wt %.

The above-mentioned "low substituted hydroxypropylcellulose" is a low substituted hydroxypropyl ether with a cellulose, which includes, for example, "low substituted hydroxypropylcellulose" which is defined in the Japanese Pharmacopoeia. The low substituted hydroxypropylcellulose includes, for example, L-HPC^{™} LH-21 (Shin-Etsu Chemical Co., Ltd.).

The "pregelatinized starches" includes, for example, "pregelatinized starch" and "partly pregelatinized starch" defined in the Japanese Pharmaceutical Excipient, and also includes "pregelatinized starch" in the USP/NF and "starch, pregelatinised" in the Ph. Eur. The "pregelatinized starches" includes preferably "partly pregelatinized starch". A commercially-available pregelatinized starch or partly pregelatinized starch includes, for example, PCS (trade name, distributor: Asahi Kasei Corporation), SWELSTAR (trade name, distributor: Asahi Kasei Corporation), STARCH 1500 and STARCH 1500G (trade name, distributor: Colorcon), and LYCATAB C (trade name, distributor: Roquette). The raw materials for pregelatinized starches include, for example, various starches such as cornstarch, potato starch, wheat starch, rice starch, and tapioca starch.

The content of the pregelatinized starches is generally 1 - 50 wt % per 100 wt % of the whole dosage form, which includes, for example, preferably 5 - 40 wt %, and more preferably 10 - 30 wt %.

### (iii) Binder

"Binder" denotes an ingredient to be used to impart binding force to powder and to form and maintain the shape of a dosage form, and a preferred binder in the present invention is a water-soluble polymer binder. The "water-soluble polymer binder" includes, for example, methylcellulose, hydroxypropylcellulose, hydroxypropylmethylcellulose, hydroxyethyl cellulose, hydroxymethyl cellulose, carboxymethyl cellulose, polyvinylpyrrolidone, polyvinyl alcohol, copolyvidone, polyethylene glycol, polyvinyl alcohol-acrylic acid-methyl methacrylate copolymer, vinyl pyrrolidone/vinyl acetate copolymer, polyvinyl alcohol-polyethylene glycol graft copolymer, pregelatinized starches, dextrin, dextran, pullulan, alginic acid, gelatin, and pectin; more preferably hydroxypropylcellulose, hydroxypropylmethylcellulose, and polyvinyl alcohol; and even more preferably hydroxypropylcellulose. The water-soluble polymer binder used herein may be one selected from those mentioned above or two or more thereof simultaneously.

The use of hydroxypropylcellulose as a water-soluble polymer binder can bring in a satisfactory preparation of drug-containing granules in formulation process (that is, granulation) and also bring in a dosage form with excellent handleability and good disintegration. In particular, a dosage form with good storage stability can be obtained in a low-content dosage form.

The viscosity of the above hydroxypropylcellulose includes, but is not limited to, 2 - 400 mPa·s, preferably 2 - 10 mPa·s.

The content of the binder is generally 1 - 20 wt % per 100 wt % of the whole dosage form, which includes, for example, preferably 1 - 10 wt %, and more preferably 1 - 5 wt %.

The binder disclosed herein can be dissolved in a solvent such as water and sprayed in granulation. In addition, it is also possible to dissolve a binder with other ingredients in a solvent such as water to be sprayed in granulation.

### (iv) Lubricant

"Lubricant" is an ingredient to be added in the preparing process of capsules or tablets for the purpose of improving the powder fluidity and fillability and preventing adhesion in filling capsules or tableting. In the case of tablets, the lubricant may be mixed with other ingredients before tableting, or may be sprayed onto the punch and die during tableting.

The lubricant includes, for example, magnesium stearate, sodium stearyl fumarate, talc, polyethylene glycol, silica, and hydrogenated vegetable oil; more preferably magnesium stearate and sodium stearyl fumarate; and even more preferably sodium stearyl fumarate. The solid dosage form disclosed herein comprises sodium stearyl fumarate as a lubricant. When sodium stearyl fumarate is used as a lubricant, it is possible to suppress tableting troubles during tableting, and to obtain a dosage form with excellent handleability and good disintegration.

The content of the lubricant is generally 0.1 - 20 wt % per 100 wt % of the whole dosage form, which includes, for example, preferably 1 - 20 wt %, more preferably 2 - 10 wt %, and even more preferably 2.5 - 10 wt %.

### (v) Excipient

"Excipient" is an ingredient to be added to make a dosage form, increase the volume, or dilute the main drug to make it easier to handle when the main drug alone does not provide sufficient "bulk" in making a dosage form, further which can bring in improvement of the powder mixability, improvement of the granulation properties when making particles in the case of granules, improvement of the fillability, adhesion, and fluidity in the die during tableting in the case of tablets, and improvement of the fillability into capsules in the case of capsules, besides simply increasing the amount.

The "excipient" includes water-soluble excipients and water-insoluble excipients, preferably water-soluble excipients.

As the water-soluble excipient used herein, a water-soluble excipient that is commonly used in formulation can be used, and preferred examples include sugars and sugar alcohols.

The sugars and sugar alcohols used herein include, but is not limited to, for example, D-mannitol, erythritol, xylitol, maltitol, sorbitol, lactose, sucrose, and trehalose; preferably D-mannitol, erythritol, lactose, and trehalose; more preferably D-mannitol and lactose; and the most preferably D-mannitol. The water-soluble excipient may be one selected from those mentioned above or a combination of two or more thereof.

The content of the excipient is generally 0.1 - 90 wt % per 100 wt % of the whole dosage form, which includes, for example, preferably 10 - 90 wt %, and more preferably 30 - 80 wt %.

### (vi) Additive

"Additive" denotes ingredients other than an active ingredient comprised in a dosage form, which is used to enhance the utility of the active ingredient and the dosage form, facilitate the formulation, stabilize the quality, or improve the usability.

The dosage form of the present invention may comprise non-toxic and inert additives commonly-used in the pharmaceutical field if necessary, as long as they do not affect the action of the present medicament. The additives include those that do not affect the therapeutic effect of the present medicament and are used in common oral preparations. The additives include, but is not limited to, for example, stabilizer, flavoring agent, sweetener, deodorant, fragrance, antioxidant, antistatic agent, fluidizer, colorant, plasticizer, antiagglomerating agent, and polish agent.

The content of the additive may be arbitrarily adjusted, which includes, for example, 0.001 - 10 wt %, preferably 0.005 - 10 wt %, and more preferably 0.01 - 5 wt % per 100 wt % of the whole dosage form. Another preferred embodiment includes a dosage form comprising no additives.

"Coating agent" denotes an ingredient for coating the surface of a dosage form, which is used to prevent the contact with water, air, and light, impart pharmaceutical characteristics such as masking of odor and bitterness, and sustained-release/enteric properties, or increase commercial value through appearance.

The coating agent includes, but is not limited to, for example, a combination of a base material such as hypromellose, hydroxypropylcellulose, polyvinylpyrrolidone, polyvinyl alcohol, polyvinyl alcohol-polyethylene glycol graft copolymer, methacrylic acid copolymer L, methacrylic acid copolymer LD, methacrylic acid copolymer S, aminoalkyl methacrylate copolymer RS, and ethyl acrylate-methyl methacrylate copolymer; and a plasticizing agent such as polyethylene glycol, propylene glycol, triacetin, triethyl citrate, glycerin, and glycerin fatty acid ester. Further, it is possible to add another additive such as titanium oxide, iron oxide, talc, and colorant to the above combination.

"Colorant" denotes an ingredient to be used for identifying capsules, tablets, etc., shielding of contained medicines from light, or adding commercial value. The colorant includes, for example, tar pigments, lake pigments, yellow ferric oxide, red ferric oxide, and titanium oxide. In addition, after film-coating, carnauba wax, talc, etc. can be added as a brightening agent.

The preparation of the oral dosage form disclosed herein varies depending on the desired dosage form, but the desired dosage form can be made according to conventional. methods.

### (1) Preparation of aqueous solution of binder:

A binder is dissolved in purified water. The amount of the binder is selected from the range of, for example, 1 - 20 wt %, preferably 2 - 8 wt % per the amount of purified water.

### (2) Preparation of granules comprising the present medicament:

The present medicament, a water-soluble excipient, and a disintegrant are charged in a granulator, and the binder solution prepared in the above step (1) is dispersed to the mixture while granulating. Alternatively, the granulation can also be carried out while spraying a solvent such as water into a granulator charged with the present medicament, a water-soluble excipient, a disintegrant and a binder.

The granulator used herein includes, but is not limited to, granulators classified as, for example, Fluid Bed Granulation, High-Share Granulation, Roto Fluid Bed Granulation, Twin Screw Granulation, etc.

### (3) Drying of granules:

The above granules are dried under reduced pressure or ambient pressure. The drying is carried out so that the loss on drying measured with an infrared moisture meter would be, for example, within 4 wt %, preferably within 2 wt %.

### (4) Addition of lubricant:

A lubricant is added to the granules dried in the above step (3) and mixed. The mixing is carried out with a mixer classified as, for example, Diffusion Mixers [Tumble]. The mixer includes, but is not limited to, for example, Tumble Blender, V Blenders, Double Cone, and Bin Tumble.

### (5) Tableting:

The above mixture is tableted to prepare tablets. The tableting machine includes, for example, tableting machines classified as Tablet Press. The tableting hardness is selected from a range of, for example, 20 - 250 N.

### (6) Optional film-coating:

The tablet prepared above may be film-coated as appropriate. The coating machine includes, for example, machines classified as coating pan. Preferred examples of the machine include machines classified as Perforated Coating System.

### (7) Drying:

The tablet prepared above is dried. The drying is carried out under reduced pressure or ambient pressure so that the loss on drying measured with an infrared moisture meter would be, for example, within 4 wt %, preferably within 2 wt %.

The present disclosure provides an oral solid dosage form comprising
(i) N²-{[1-ethyl-6-(4-methylphenoxy)-1H-benzimidazol-2-yl]methyl}-L-alaninamide or a pharmaceutically acceptable salt thereof, or a hydrate or solvate thereof,
(ii) a disintegrant comprising pregelatinized starches, and
(iii) hydroxypropylcellulose as a water-soluble polymer binder,
further optionally comprising (iv) an excipient, (v) a lubricant, and/or (vi) an additive, as appropriate.

The present disclosure provides an oral solid dosage form comprising
(i) N²-{[1-ethyl-6-(4-methylphenoxy)-1H-benzimidazol-2-yl)methyl}-L-alaninamide or a pharmaceutically acceptable salt thereof, or a hydrate or solvate thereof,
(ii) a disintegrant comprising pregelatinized starches and low substituted hydroxypropylcellulose, and
(iii) hydroxypropylcellulose as a water-soluble polymer binder,
further optionally comprising (iv) an excipient, (v) a lubricant, and/or (vi) an additive, as appropriate.

### [2] Medical use of oral solid dosage form

The present medicament selectively inhibits voltage-gated sodium (Na⁺) channel current in human Nav1.7 and Nav1.8 expressed mainly in peripheral nerves, thereby which is effective as a medicament for treating and preventing pain diseases. Thus, the present invention relates to a pharmaceutical composition for treating and/or preventing pain diseases comprising the present medicament, an agent for treating and/or preventing pain diseases, preferably an oral solid dosage form.

The "prevention" used herein is the act of administering the present medicament which is an active ingredient to healthy individuals who have not developed a disease or are in good health at the time of the administration. And, the "agent for the prevention" used herein is administered to such healthy individuals, for example, whose purpose is to prevent the development of the disease, i.e., it is expected to be appropriate for people who have had previous symptoms of the disease or who are considered to be at increased risk of contracting the disease. The "treatment" used herein is the act of administering the present medicament which is an active ingredient to individuals (patients) who have been diagnosed by a doctor as having a disease. And, the "agent for the treatment" used herein is administered to such patients, for example, whose purpose is to alleviate a disease or symptom, not to exacerbate a disease or symptom, or to return to the state before the onset of a disease. Even if the purpose of administration is to prevent aggravation of a disease or condition, if it is administered to a patient, it is a therapeutic act.

The "pain" used herein means a pain felt in the peripheral nerves, which includes a pain associated with peripheral nerves such as C fibers and Aδ fibers; a spontaneous pain such as numbness, burning sensation, dull pain, stinging pain, and electric-shock-like pain; a neuropathic pain; a nociceptive pain; and an inflammatory pain. The neuropathic pain includes, for example, peripheral neuropathic pain, neuropathic pain with hyperalgesia or allodynia to mechanical or cold stimuli, diabetic neuropathy, postherpetic neuralgia, chemotherapy-induced neuropathy, cancer pain, sensory nerve damage caused by viral infection in human immunodeficiency syndrome, trigeminal neuralgia, complex regional pain syndrome, reflex sympathetic dystrophy, neuralgia after low back surgery, phantom limb pain, pain after spinal cord injury, persistent postoperative pain, inflammatory demyelinating polyradiculoneuropathy, alcoholic neuropathy, entrapment peripheral neuropathy, carpal tunnel syndrome, cubital tunnel syndrome, tarsal tunnel syndrome, iatrogenic neuropathy, sudden sensorineural disorder, malnutrition-induced neuropathy, radiation-induced neuropathy, radiculopathy, toxic peripheral neuropathy, traumatic peripheral neuropathy, brachial plexus avulsion injury, glossopharyngeal neuralgia, autoimmune neuropathy, chronic cauda equina disorder, small fiber neuropathy, erythromelalgia, paroxysmal extreme pain disorder, and pediatric extremity pain attack. The nociceptive pain or inflammatory pain includes low back pain, back pain, abdominal pain, chronic rheumatoid arthritis, pain due to osteoarthritis, muscular pain, acute postoperative pain, post-extraction pain, fracture pain, burn pain, and pain associated with UV irradiation. In addition, the present compound can be used as a medicament for the treatment or prevention of dysuria. The term "dysuria" used herein includes frequent urination, and bladder pain caused by prostatic hyperplasia.

For the enhancement of its action, the present medicament may be used in combination with, for example, a non-steroidal anti-inflammatory agent such as celecoxib, ibuprofen, loxoprofen, acetaminophen, and diclofenac; a steroidal anti-inflammatory agent such as dexamethasone and prednisolone; and an opioid analgesic agent such as tramadol, morphine, oxycodone, hydrocodone, and fentanyl; a sodium channel blocker such as lidocaine, bupivacaine, and propitocaine; and an analgesic agent such as adrenaline, in order to enhance the action of the present medicament. The present medicament may be also used in combination with an antiepileptic agent such as pregabalin and carbamazepine; an aldose reductase inhibitor such as epalrestat; an prostaglandin derivative drug such as limaprost alfadex; an antidepressant agent such as amitriptyline and duloxetine; an anticonvulsant agent; an antianxiety agent; a dopamine receptor agonist; an antiparkinsonian agent; a hormone preparation; a migraine medication; a beta-adrenergic receptor antagonist; an anti-dementia agent; and an mood-disorder amelioration agent. A more preferred agent combined with the present medicament includes an antiepileptic agent such as pregabalin and carbamazepine; an antidepressant agent such as amitriptyline and duloxetine; a narcotic analgesic agent such as morphine, oxycodone, and tramadol; an anti-inflammatory agent such as acetaminophen, diclofenac, and dexamethasone; an aldose reductase inhibitor such as epalrestat; and a prostaglandin derivative such as limaprost alfadex. The present compound may also be combined with an agent such as an antiemetic agent and a sleep-inducing agent in order to reduce side effects.

The present medicament and another agent for the combination use may be administered at any appropriate time, and may be administered to a subject of the treatment at the same time, or with any appropriate intervals. The present medicament may be formulated into a single unit dosage form with the other agent for the combination, or the present medicament and the other agent for the combination are administered in separate formulations or via separate administration routes. The dose of the other agent for the combination may be suitably chosen on the basis of the dose for clinical use. The ratio of the present medicament and the other agent for the combination may be suitably chosen depending on the subject of the treatment, the route of administration, the targeted disease, the symptoms, or combination thereof.

The administration route of the present compound or a pharmaceutically acceptable salt thereof, or a hydrate or solvate thereof may be either oral or parenteral administration, but oral administration is preferable. The dosage of the present compound may vary depending on the administration route, the symptom and age of patients, etc., which includes a range of, generally 0.01 - 30 mg/kg/day, preferably 0.05 - 10 mg/kg/day, more preferably 0.1 - 3 mg/kg/day. In another embodiment, the dosage includes a range of, generally 0.01 mg - 1000 mg/day, preferably 0.1 mg - 500 mg/day, more preferably 0.5 mg - 300 mg/day, even more preferably 1 mg - 200 mg/day, the most preferably 5 mg - 100 mg/day. The administration frequency is preferably once a day or several times a day, for example, preferably 2 or 3 times a day.

### EXAMPLES

Hereinafter, the present invention is illustrated with Examples, but the present invention is not limited thereto. The names of compounds in the following Examples, Tests, and Reference examples do not necessarily conform to the IUPAC nomenclature.

In Examples, Tests, and Reference examples, "%" used in a solution denotes "w/w %", and "%" used in a particle denotes "wt %", unless otherwise indicated.

The additives used in Examples, Tests, and Reference examples were the followings, unless otherwise indicated.
hydroxypropylcellulose: HPC-SL (3 - 5.9 mPa·s¹⁾) (NIPPON SODA CO., LTD.)
hydroxypropylcellulose: HPC-L (6 - 10 mPa·s¹⁾) (NIPPON SODA CO., LTD.)
hypromellose (hydroxypropylmethylcellulose) (TC-5E^{™}/3 mPa·s, Tc-5R^{™}/6 mPa·s²⁾): Shin-Etsu Chemical Co., Ltd.
polyvinyl alcohol (Gohsenol^{™} EG-05P/4.8 - 5.8 mPa·s³⁾): Mitsubishi Chemical Corporation
partly pregelatinized starch: PCS^{™} (trade name, distributor: Asahi Kasei Corporation)
low substituted hydroxypropylcellulose: L-HPC^{™} LH-21 (Shin-Etsu Chemical Co., Ltd.)
sodium carboxymethyl starch: Primojel (DMV)
croscarmellose sodium: Ac-Di-Sol^{™} (FMC BioPolymer)
mannitol: Mannit P (Mitsubishi Corporation Life Sciences Limited)
sodium stearyl fumarate: PRUV^{™} (JRS PHARMA)
OPADRY^{™} 03H430000 ORANGE: Colorcon

The OPADRY used in the following examples is a film-coating agent which does not affect the dissolution property.
1) Maker's catalog value in 20°C, 2 % solution
2) Maker's catalog value in 20°C, 2 % solution
3) Maker's catalog value in 20°C, 4 % solution

### Example 1

Film-coated tablet comprising 5 mg of the present compound, wherein hydroxypropylcellulose is used as a binder, and partly pregelatinized starch and low substituted hydroxypropylcellulose are used as disintegrants (drug concentration: 1.8 %)
According to the following (1-1), (1-2), (1-3), (1-4), and (1-5), a granule, a mixed powder, an uncoated tablet, and a film-coated tablet were prepared in order, and a storage stability test (stress test) of the film-coated tablet was carried out.

The used amounts shown in parentheses in the following description are each one example for preparing the formulation shown in Example 1. Theoretically, when this preparing method is followed, even if the used amounts are changed at the same ratio and the same procedure is followed, the same preparation can be performed.

### (1-1) Preparation of binding solution (5 % aqueous hydroxypropylcellulose) :

Hydroxypropylcellulose (42 g) as a binder was dissolved in purified water (798 g) to prepare a binding solution.

### (1-2) Granulation:

The present compound (25 g), mannitol (914 g), partly pregelatinized starch (175 g), and low substituted hydroxypropylcellulose (175 g) were thrown in a fluidized bed granulator/dryer machine (Multiplex FD-MP-01/POWREX CORPORATION), and the mixture was granulated by spraying the binding solution prepared in the above (1-1) under the condition defined below to prepare a granule. The obtained granule was mixed with sodium stearyl fumarate to prepare a mixed powder for low drug-concentration (the formula for low drug-concentration is collectively shown in Table 1 below as "Formula A"). Here, the amount of sodium stearyl fumarate to be used was calculated from Formula A based on the amount for preparing the granule shown in Formula A, then which was mixed with the granule.

### Granulation condition

Charge air temperature: 75°C
Charge air volume: 50 m³/hr
Spray speed: 9 - 14 g/min
Spray nozzle diameter: 1.0 mm
Spray pressure: 0.2 MPa
Spray type: top-spray

### (1-3) Tableting:

The mixed powder prepared in the above (1-2) was tableted with a rotary tablet press VEL2 0308SW2M (KIKUSUI SEISAKUSHO LTD.) to prepare an uncoated tablet.
Punch size: 9 mm
Compression pressure for tableting: 10 kN

### (1-4) Coating:

To purified water in a SUS beaker were added hypromellose Tc-5R^{™} (9.36 g) and propylene glycol (2.16 g) while stirring, and the mixture was dissolved to give film-coating solution (1). To purified water in another SUS beaker were gradually added titanium oxide (2.30 g) and red ferric oxide (0.58 g), and the mixture was dispersed to give film-coating solution (2). Film-coating solution (1) and film-coating solution (2) were mixed, and purified water was added thereto until it reaches 273.6 g. The solution was passed through a screen with an opening of 105 µm to give film-coating solution (3).

Film-coating solution (3) was sprayed to the uncoated tablet prepared in the above (1-3) in a pan coating machine FC-HI-COATER HCT-30N (Freund Corp.) under the condition defined below so that the coating amount was 6.0 mg to give a film-coated tablet.

### Condition for film-coating

Charge air temperature: 85°C
Charge air volume: 0.6 m³/hr
Spray pressure: 0.16 MPa
Liquid velocity: 5 - 6 g/min

### (1-5) Storage stability test:

The film-coated tablet prepared in the above (1-4) was stored under the condition defined in Test 2 shown below.

### Example 2

Uncoated tablet comprising 5 mg of the present invention, wherein hydroxypropylcellulose is used as a binder, and partly pregelatinized starch is used as a disintegrant (drug concentration: 1.8 %)
According to the following (2-1), (2-2), (2-3), and (2-4), a granule, a mixed powder, and an uncoated tablet were prepared in order, and a storage stability test (stress test) of the uncoated tablet was carried out.

The used amounts shown in parentheses in the following description are each one example for preparing the formulation shown in Example 2. Theoretically, when this preparing method is followed, even if the used amounts are changed at the same ratio and the same procedure is followed, the same preparation can be performed.

### (2-1) Preparation of binding solution (5 % aqueous hydroxypropylcellulose):

Hydroxypropylcellulose (3.4 g) as a binder was dissolved in purified water (64.64 g) to prepare a binding solution.

### (2-2) Granulation:

The present compound (2.03 g), mannitol (76.22 g), and partly pregelatinized starch (28.35 g) were thrown in a fluidized bed granulator/dryer machine (FL-LABO/Freund Corporation), and the mixture was granulated by spraying the binding solution prepared in the above (2-1) under the condition defined below to prepare a granule. The obtained granule was mixed with sodium stearyl fumarate to prepare a mixed powder for low drug-concentration (the formula for low drug-concentration is collectively shown in Table 1 below as "Formula A"). Here, the amount of sodium stearyl fumarate to be used was calculated from Formula A based on the amount for preparing the granule shown in Formula A, then which was mixed with the granule.

### Granulation condition

Charge air temperature: 75°C
Charge air volume: 0.1 - 0.2 m³/min
Spray speed: 3 - 5 g/min
Spray nozzle diameter: 1.0 mm
Spray pressure: 0.12 MPa

### (2-3) Tableting:

The mixed powder prepared in the above (2-2) was tableted with a rotary tablet press VEL2 0308SW2M (KIKUSUI SEISAKUSHO LTD.) to prepare an uncoated tablet.
Punch size: 9 mm
Compression pressure for tableting: 10 kN

### (2-4) Storage stability test:

The uncoated tablet prepared in the above (2-3) was stored under the condition defined in Test 2 shown below.

### Example 3

Uncoated tablet comprising 5 mg of the present invention, wherein hydroxypropylcellulose is used as a binder, and partly pregelatinized starch and low substituted hydroxypropylcellulose are used as disintegrants (drug concentration: 1.8 %)
According to the following (3-1), (3-2), (3-3), and (3-4), a granule, a mixed powder, and an uncoated tablet were prepared in order, and a storage stability test (stress test) of the uncoated tablet was carried out.

The used amounts shown in parentheses in the following description are each one example for preparing the formulation shown in Example 3. Theoretically, when this preparing method is followed, even if the used amounts are changed at the same ratio and the same procedure is followed, the same preparation can be performed.

### (3-1) Preparation of binding solution (5 % aqueous hydroxypropylcellulose) :

Hydroxypropylcellulose (3.4 g) as a binder was dissolved in purified water (64.64 g) to prepare a binding solution.

### (3-2) Granulation:

The present compound (2.03 g), mannitol (76.22 g), partly pregelatinized starch (28.35 g), and low substituted hydroxypropylcellulose (14.18 g) were thrown in a fluidized bed granulator/dryer machine (FL-LABO/Freund Corporation), and the mixture was granulated by spraying the binding solution prepared in the above (3-1) under the condition defined below to prepare a granule. The obtained granule was mixed with sodium stearyl fumarate to prepare a mixed powder for low drug-concentration (the formula for low drug-concentration is collectively shown in Table 1 below as "Formula A"). Here, the amount of sodium stearyl fumarate to be used was calculated from Formula A based on the amount for preparing the granule shown in Formula A, then which was mixed with the granule.

### Granulation condition

Charge air temperature: 75°C
Charge air volume: 0. - 0.3 m³/min
Spray speed: 4 - 5 g/min
Spray nozzle diameter: 1.0 mm
Spray pressure: 0.12 MPa

### (3-3) Tableting:

The mixed powder prepared in the above (3-2) was tableted with a rotary tablet press VEL2 0308SW2M (KIKUSUI SEISAKUSHO LTD.) to prepare an uncoated tablet.
Punch size: 9 mm
Compression pressure for tableting: 10 kN

### (3-4) Storage stability test:

The uncoated tablet prepared in the above (3-3) was stored under the condition defined in Test 2 shown below.

### Reference example 1

Uncoated tablet comprising 5 mg of the present invention, wherein hydroxypropylcellulose is used as a binder, partly pregelatinized starch and sodium carboxymethyl starch are used as disintegrants, and sodium stearyl fumarate is used as a lubricant (drug concentration: 1.8 %)
According to the following (4-1), (4-2), (4-3), and (4-4), a granule, a mixed powder, and an uncoated tablet were prepared in order, and a storage stability test (stress test) of the uncoated tablet was carried out.

The used amounts shown in parentheses in the following description are each one example for preparing the formulation shown in Reference example 1. Theoretically, when this preparing method is followed, even if the used amounts are changed at the same ratio and the same procedure is followed, the same preparation can be performed.

### (4-1) Preparation of binding solution (5 % aqueous hydroxypropylcellulose):

Hydroxypropylcellulose (3.4 g) as a binder was dissolved in purified water (64.64 g) to prepare a binding solution.

### (4-2) Granulation:

The present compound (2.03 g), mannitol (76.22 g), partly pregelatinized starch (14.18 g), and sodium carboxymethyl starch (14.18 g) were thrown in a fluidized bed granulator/dryer machine (FL-LABO/Freund Corporation), and the mixture was granulated by spraying the binding solution prepared in the above (4-1) under the condition defined below to prepare a granule. The obtained granule was mixed with sodium stearyl fumarate to prepare a mixed powder for low drug-concentration (the formula for low drug-concentration is collectively shown in Table 1 below as "Formula A"). Here, the amount of sodium stearyl fumarate to be used was calculated from Formula A based on the amount for preparing the granule shown in Formula A, then which was mixed with the granule.

### Granulation condition

Charge air temperature: 75°C
Charge air volume: 0.2 m³/min
Spray speed: 3 - 4 g/min
Spray nozzle diameter: 1.0 mm
Spray pressure: 0.12 MPa

### (4-3) Tableting:

The mixed powder prepared in the above (4-2) was tableted with a rotary tablet press VEL2 0308SW2M (KIKUSUI SEISAKUSHO LTD.) to prepare an uncoated tablet.
Punch size: 9 mm
Compression pressure for tableting: 10 kN

### (4-4) Storage stability test

The uncoated tablet prepared in the above (4-3) was stored under the condition defined in Test 2 shown below.

### Formula A (Formulae of uncoated tablets in Examples 1, 2, and 3, and Reference example 1: drug concentration 1.8 %)

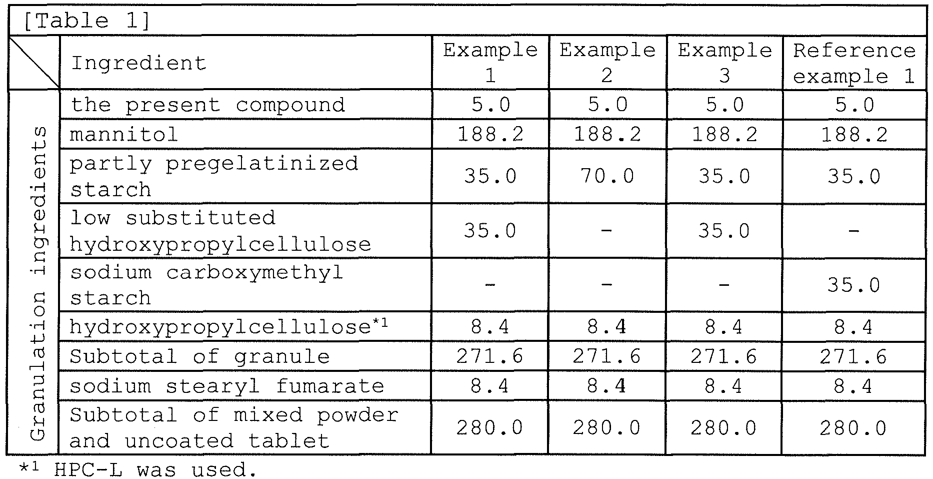

The formulations prepared above were evaluated about their quality in the following manner.

### <Test 1>

Table 2 shows the evaluation results of the presence or absence of tableting troubles in tableting of Examples 1, 2, and 3 and Reference example 1. Specifically, the evaluation was made under the following conditions.

Presence or absence of tableting troubles: The presence or absence of tableting troubles such as sticking and die friction during tableting, and the extent to which it affects the appearance of tablets as products were evaluated.

| [Table 2] | | | | |
|---|---|---|---|---|
| | Example 1 | Example 2 | Example 3 | Ref. example 1 |
| Drug concentration (%) | 1.8 | 1.8 | 1.8 | 1.8 |
| Tableting trouble | No | No | No | No |

| | | | | |
|---|---|---|---|---|
| No: No tableting trouble that affects product appearance | | | | |

### <Test 2>

### Storage stability test (purity test)

Each uncoated tablet or film-coated tablet which were prepared in Examples 1, 2, and 3, and Reference example 1 were put in an amber screw-cap test tube (material: glass), and each test tube in open state was stored at 50°C/85 % RH in a thermo-hygrostat container for two weeks or four weeks. Each sample was purity-tested to evaluate the amount of the produced impurity.

### Test conditions

Detector: UV absorption photometer (wavelength for measurement 220 nm)
Column: in which a stainless tube with an inner diameter of 4.6 mm and a length of about 10 cm is filled with 3 µm octadecylsilylated silica gel for liquid chromatography (YMC-Pack Pro C18 RS).
Column temperature: constant temperature around 40°C
Mobile phase A: 0.01 mol/L phosphate buffer
Mobile phase B: methanol/tetrahydrofuran mixture (4:1)
Elution method: high-pressure gradient method
Flow rate: about 1 mL/min
Time of measuring area: for 70 minutes after injection
Impurity 1: an impurity detected at a relative retention time of about 0.45 when measured under the above conditions.

Based on each peak area obtained by liquid chromatography, the amount of Impurity 1 relative to the present compound was calculated. Table 3 shows the results.

| [Table 3] | | | | | |
|---|---|---|---|---|---|
| | | Example 1 | Example 2 | Example 3 | Ref. example 1 |
| Drug concentration (%) | | 1.8 | 1.8 | 1.8 | 1.8 |
| Amount of Impurity 1 (%) | Before storage | 0.00 | 0.00 | 0.00 | 0.00 |
| | 50°C/85%RH 2 weeks later | 0.03 | 0.04 | 0.06 | 0.54 |
| | 50°C/85%RH 4 weeks later | 0.09 | 0.09 | 0.14 | 1.21 |

### Example 4

Uncoated tablet comprising 5 mg of the present invention, wherein hydroxypropylcellulose is used as a binder, partly pregelatinized starch is used as a disintegrant, and sodium stearyl fumarate is used as a lubricant (drug concentration: 7.1 %)

According to the following (5-1), (5-2), (5-3), and (5-4), a granule, a mixed powder, and an uncoated tablet were prepared in order, and a storage stability test (stress test) of the uncoated tablet was carried out.

The used amounts shown in parentheses in the following description are each one example for preparing the formulation shown in Example 4. Theoretically, when this preparing method is followed, even if the used amounts are changed at the same ratio and the same procedure is followed, the same preparation can be performed.

### (5-1) Preparation of binding solution (5 % aqueous hydroxypropylcellulose):

Hydroxypropylcellulose (15 g) as a binder was dissolved in purified water (285 g) to prepare a binding solution.

### (5-2) Granulation:

The present compound (50 g), mannitol (535 g), and partly pregelatinized starch (100 g) were thrown in a fluidized bed granulator/dryer machine (Multiplex FD-MP-01/POWREX CORPORATION), and the mixture was granulated by spraying the binding solution prepared in the above (5-1) under the condition defined below to prepare a granule. The obtained granule was mixed with sodium stearyl fumarate to prepare a mixed powder for middle drug-concentration (the formula for middle drug-concentration is collectively shown in Table 4 below as "Formula B"). Here, the amount of sodium stearyl fumarate to be used was calculated from Formula B based on the amount for preparing the granule shown in Formula B, then which was mixed with the granule.

### Granulation condition

Charge air temperature: 75 - 80°C
Charge air volume: 50 - 55 m³/hr
Spray speed: 10 - 15 g/min
Spray nozzle diameter: 1.0 mm
Spray pressure: 0.12 MPa
Spray type: top-spray

### (5-3) Tableting:

The mixed powder prepared in the above (5-2) was tableted with a rotary tablet press HT-AP12SS-II (HATA TEKKOSHO) to prepare an uncoated tablet.
Punch size: φ 5.5 mm7R
Compression pressure for tableting: 4 kN

### (5-4) storage stability test:

The uncoated tablet prepared in the above (5-3) was stored under the condition defined in Test 2 shown above.

### Example 5

Film-coated tablet comprising 5 mg of the present compound, wherein hydroxypropylcellulose is used as a binder, and partly pregelatinized starch and low substituted hydroxypropylcellulose are used as disintegrants (drug concentration: 7.1 %)
According to the following (6-1), (6-2), (6-3), (6-4), and (6-5), a granule, a mixed powder, an uncoated tablet, and a film-coated tablet were prepared in order, and a storage stability test (stress test) of the film-coated tablet was carried out.

The used amounts shown in parentheses in the following description are each one example for preparing the formulation shown in Example 5. Theoretically, when this preparing method is followed, even if the used amounts are changed at the same ratio and the same procedure is followed, the same preparation can be performed.

### (6-1) Preparation of binding solution (5 % aqueous hydroxypropylcellulose) :

Hydroxypropylcellulose (42 g) as a binder was dissolved in purified water (798 g) to prepare a binding solution.

### (6-2) Granulation:

The present compound (100 g), mannitol (866 g), partly pregelatinized starch (175 g), and low substituted hydroxypropylcellulose (175 g) were thrown in a fluidized bed granulator/dryer machine (Multiplex FD-MP-01/POWREX CORPORATION), and the mixture was granulated by spraying the binding solution prepared in the above (6-1) under the condition defined below to prepare a granule. The obtained granule was mixed with sodium stearyl fumarate to prepare a mixed powder for middle drug-concentration (the formula for middle drug-concentration is collectively shown in Table 4 below as "Formula B"). Here, the amount of sodium stearyl fumarate to be used was calculated from Formula B based on the amount for preparing the granule shown in Formula B, then which was mixed with the granule.

### Granulation condition

Charge air temperature: 80 - 90°C
Charge air volume: 55 - 65 m³/hr
Spray speed: 12 - 13 g/min
Spray nozzle diameter: 1.0 mm
Spray pressure: 0.15 MPa
Spray type: top-spray

### (6-3) Tableting:

The mixed powder prepared in the above (6-2) was tableted with a rotary tablet press VEL2 0308SW2M (KIKUSUI SEISAKUSHO LTD.) to prepare an uncoated tablet.
Punch size: φ 5.5 mm7R
Compression pressure for tableting: 6 kN

### (6-4) Coating:

To 1800 g of purified water in a SUS beaker was gradually added 200 g of OPADRY^{™} 03H43000 ORANGE while stirring, and the mixture was dispersed and dissolved. The solution was passed through a screen with an opening of 105 µm to give film-coating solution (4).

Film-coating solution (4) was sprayed to the uncoated tablet prepared in the above (6-3) in a pan coating machine FC-HI-COATER HCT-30N (Freund Corp.) under the condition defined below so that the coating amount was 2.5 mg to give a film-coated tablet.

### Condition for film-coating

Charge air temperature: 80°C
Charge air volume: 0.6 m³/hr
Spray pressure: 0.15 MPa
Liquid velocity: 5 g/min

### (6-5) Storage stability test:

The film-coated tablet prepared in the above (6-4) was stored under the condition defined in Test 2 shown above.

### Example 6

Film-coated tablet comprising 10 mg of the present compound, wherein hydroxypropylcellulose is used as a binder, and partly pregelatinized starch and low substituted hydroxypropylcellulose are used as disintegrants (drug concentration: 14.3 %)
According to the following (7-1), (7-2), (7-3), (7-4), and (7-5), a granule, a mixed powder, an uncoated tablet, and a film-coated tablet were prepared in order, and a storage stability test (stress test) of the film-coated tablet was carried out.

The used amounts shown in parentheses in the following description are each one example for preparing the formulation shown in Example 6. Theoretically, when this preparing method is followed, even if the used amounts are changed at the same ratio and the same procedure is followed, the same preparation can be performed.

### (7-1) Preparation of binding solution (5 % aqueous hydroxypropylcellulose) :

Hydroxypropylcellulose (42 g) as a binder was dissolved in purified water (798 g) to prepare a binding solution.

### (7-2) Granulation:

The present compound (200 g), mannitol (766 g), partly pregelatinized starch (175 g), and low substituted hydroxypropylcellulose (175 g) were thrown in a fluidized bed granulator/dryer machine (Multiplex FD-MP-01D/POWREX CORPORATION), and the mixture was granulated by spraying the binding solution prepared in the above (7-1) under the condition defined below to prepare a granule. The obtained granule was mixed with sodium stearyl fumarate in a Type V mixer (Tsutsui Rikagaku Kikai) at 40 rpm for 30 minutes to prepare a mixed powder for middle drug-concentration (the formula for middle drug-concentration is collectively shown in Table 4 below as "Formula B"). Here, the amount of sodium stearyl fumarate to be used was calculated from Formula B based on the amount for preparing the granule shown in Formula B, then which was mixed with the granule.

### Granulation condition

Charge air temperature: 75 - 80°C
Charge air volume: 0.5 - 0.9 m³/min
Spray speed: 10 - 14 g/min
Spray nozzle diameter: 1.0 mm
Spray pressure: 0.1 MPa
Spray type: top-spray

### (7-3) Tableting:

The mixed powder prepared in the above (7-2) was tableted with a rotary tablet press VELAC (KIKUSUI SEISAKUSHO LTD.) to prepare an uncoated tablet.
Punch size: φ 5.5 mm7R
Compression pressure for tableting: 5-6 kN

### (7-4) Coating:

To 1300 g of purified water in a SUS beaker was gradually added 150 g of OPADRY ^{™} 03H43000 ORANGE while stirring, and the mixture was dispersed and dissolved. The solution was passed through a nylon cloth with an opening of 105 µm to give film-coating solution (5).

Film-coating solution (5) was sprayed to the uncoated tablet prepared in the above (7-3) in a pan coating machine HC-LABO (Freund Corp.) under the condition defined below so that the coating amount was 2.5 mg to give a film-coated tablet.

### Condition for film-coating

Charge air temperature: 80°C
Charge air volume: 0.6 m³/hr
Spray pressure: 0.15 MPa
Liquid velocity: 3 - 5 g/min

### (7-5) Storage stability test:

The film-coated tablet prepared in the above (7-4) was stored under the condition defined in Test 2 shown above.

### Reference example 2

Uncoated tablet comprising 5 mg of the present compound, wherein hydroxypropylcellulose is used as a binder, partly pregelatinized starch is used as a disintegrant, and magnesium stearate is used as a lubricant (drug concentration: 7.1 %)
According to the following (8-1), (8-2), and (8-3), a granule, a mixed powder, and an uncoated tablet were prepared in order, and a storage stability test (stress test) of the uncoated tablet was carried out.

The used amounts shown in parentheses in the following description are each one example for preparing the formulation shown in Reference example 2. Theoretically, when this preparing method is followed, even if the used amounts are changed at the same ratio and the same procedure is followed, the same preparation can be performed.

### (8-1) Granulation:

The granule prepared in the above (5-2) was mixed with magnesium stearate to prepare a mixed powder for middle drug-concentration (the formula for middle drug-concentration is collectively shown in Table 4 below as "Formula B"). Here, the amount of magnesium stearate to be used was calculated from Formula B based on the amount for preparing the granule shown in Formula B, then which was mixed with the granule.

### (8-2) Tableting:

The mixed powder prepared in the above (8-1) was tableted with a rotary tablet press HT-AP12SS-II (HATA TEKKOSHO) to prepare an uncoated tablet.
Punch size: φ 5.5 mm7R
Compression pressure for tableting: 4 kN

### (8-3) Storage stability test:

The uncoated tablet prepared in the above (8-2) was stored under the condition defined in Test 2 shown above.

### Reference example 3

Uncoated tablet comprising 5 mg of the present compound, wherein polyvinyl alcohol is used as a binder, partly pregelatinized starch is used as a disintegrant, and sodium stearyl fumarate is used as a lubricant (drug concentration: 7.1 %)
According to the following (9-1), (9-2), (9-3), and (9-4), a granule, a mixed powder, and an uncoated tablet were prepared in order, and a storage stability test (stress test) of the uncoated tablet was carried out.

The used amounts shown in parentheses in the following description are each one example for preparing the formulation shown in Reference example 3. Theoretically, when this preparing method is followed, even if the used amounts are changed at the same ratio and the same procedure is followed, the same preparation can be performed.

### (9-1) Preparation of binding solution (5 % aqueous polyvinyl alcohol):

Polyvinyl alcohol (15 g) as a binder was dissolved in purified water (285 g) to prepare a binding solution.

### (9-2) Granulation:

The present compound (50 g), mannitol (535 g), and partly pregelatinized starch (100 g) were thrown in a fluidized bed granulator/dryer machine (Multiplex FD-MP-01/POWREX CORPORATION), and the mixture was granulated by spraying the binding solution prepared in the above (9-1) under the condition defined below to prepare a granule. The obtained granule was mixed with sodium stearyl fumarate to prepare a mixed powder for middle drug-concentration (the formula for middle drug-concentration is collectively shown in Table 4 below as "Formula B"). Here, the amount of sodium stearyl fumarate to be used was calculated from Formula B based on the amount for preparing the granule shown in Formula B, then which was mixed with the granule.

### Granulation condition

Charge air temperature: 75 - 80°C
Charge air volume: 50 - 55 m³/hr
Spray speed: 10 - 15 g/min
Spray nozzle diameter: 1.0 mm
Spray pressure: 0.12 MPa
Spray type: top-spray

### (9-3) Tableting:

The mixed powder prepared in the above (9-2) was tableted with a rotary tablet press HT-AP12SS-II (HATA TEKKOSHO) to prepare an uncoated tablet.
Punch size: φ 5.5 mm7R
Compression pressure for tableting: 4 kN

### (9-4) Storage stability test:

The uncoated tablet prepared in the above (9-3) was stored under the condition defined in Test 2 shown above.

### Reference example 4

Uncoated tablet comprising 5 mg of the present compound, wherein hypromellose is used as a binder, partly pregelatinized starch is used as a disintegrant, and sodium stearyl fumarate is used as a lubricant (drug concentration: 7.1 %)
According to the following (10-1), (10-2), (10-3), and (10-4), a granule, a mixed powder, and an uncoated tablet were prepared in order, and a storage stability test (stress test) of the uncoated tablet was carried out.

The used amounts shown in parentheses in the following description are each one example for preparing the formulation shown in Reference example 4. Theoretically, when this preparing method is followed, even if the used amounts are changed at the same ratio and the same procedure is followed, the same preparation can be performed.

### (10-1) Preparation of binding solution (5 % aqueous hypromellose):

Hypromellose TC-5E^{™} (15 g) as a binder was dissolved in purified water (285 g) to prepare a binding solution.

### (10-2) Granulation:

The present compound (50 g), mannitol (535 g), and partly pregelatinized starch (100 g) were thrown in a fluidized bed granulator/dryer machine (Multiplex FD-MP-01/POWREX CORPORATION), and the mixture was granulated by spraying the binding solution prepared in the above (10-1) under the condition defined below to prepare a granule. The obtained granule was mixed with sodium stearyl fumarate to prepare a mixed powder for middle drug-concentration (the formula for middle drug-concentration is collectively shown in Table 4 below as "Formula B"). Here, the amount of sodium stearyl fumarate to be used was calculated from Formula B based on the amount for preparing the granule shown in Formula B, then which was mixed with the granule.

### Granulation condition

Charge air temperature: 75 - 80°C
Charge air volume: 50 - 55 m³/hr
Spray speed: 10 - 15 g/min
Spray nozzle diameter: 1.0 mm
Spray pressure: 0.12 MPa
Spray type: top-spray

### (10-3) Tableting:

The mixed powder prepared in the above (10-2) was tableted with a rotary tablet press HT-AP12SS-II (HATA TEKKOSHO) to prepare an uncoated tablet.
Punch size: φ 5.5 mm7R
Compression pressure for tableting: 4 kN

### (10-4) Storage stability test:

The uncoated tablet prepared in the above (10-3) was stored under the condition defined in Test 2 shown above.

### Formula B (Formulae of uncoated tablets in Examples 4, 5, and 6, and Reference examples 2, 3, and 4: drug concentrations 7.1 %, 14.9 %)

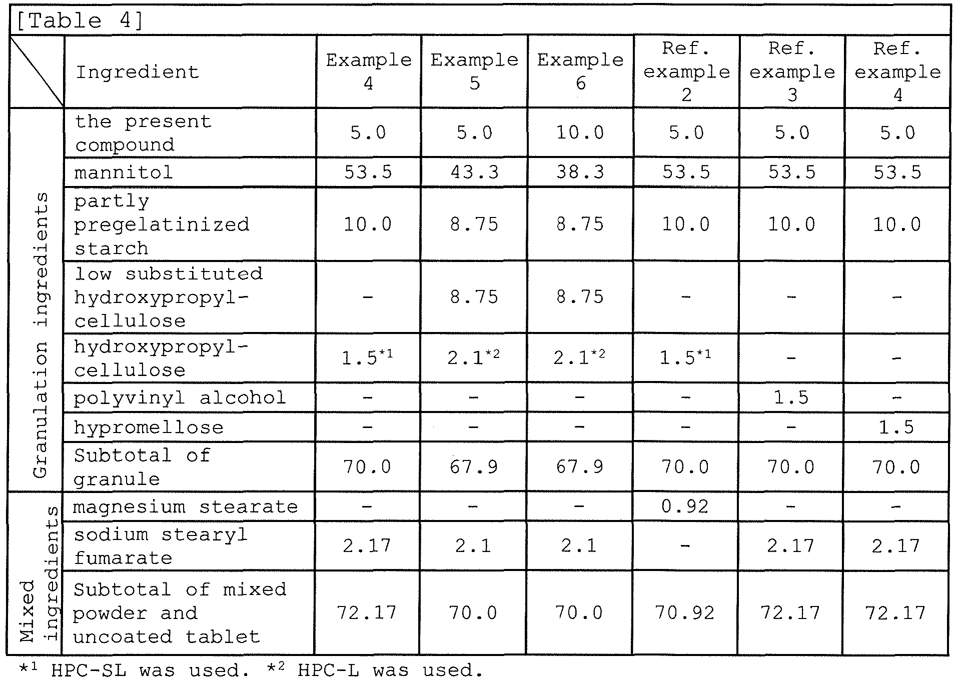

The formulations prepared above were evaluated about their quality in the following manner.

Table 5 shows the evaluation results of the presence or absence of tableting troubles in tableting of Examples 4, 5, and 6 and Reference example 2, 3, and 4. The specific evaluation was made in the manner of <Test 1> mentioned above.

| [Table 5] | | | | | | |
|---|---|---|---|---|---|---|
| | Example 4 | Example 5 | Example 6 | Ref. example 2 | Ref. example 3 | Ref. example 4 |
| Drug concentration (%) | 7.1 | 7.1 | 14.3 | 7.1 | 7.1 | 7.1 |
| Tableting trouble | No | No | No | Yes (die friction, sticking) | No | No |

| | | | | | | |
|---|---|---|---|---|---|---|
| No: No tableting trouble that affects product appearance Yes: Tableting trouble that affects product appearance | | | | | | |

### Storage stability test (purity test)

Each uncoated tablet or film-coated tablet which were prepared in Examples 4, 5, and 6, and Reference examples 2, 3, and 4 was subjected to the storage stability test (purity test) in the manner of above Test 2.

Based on each peak area obtained by liquid chromatography, the amount of Impurity 1 relative to the present compound was calculated. Table 6 shows the results.

| [Table 6] | | | | | | | |
|---|---|---|---|---|---|---|---|
| | | Example 4 | Example 5 | Example 6 | Ref. example 2 | Ref. example 3 | Ref. example 4 |
| Drug concentration (%) | | 7.1 | 7.1 | 14.3 | 7.1 | 7.1 | 7.1 |
| Amount of Impurity 1 (%) | Before storage | 0.00 | 0.00 | 0.00 | 0.00 | 0.00 | 0.00 |
| | 50°C/85%RH 2 weeks later | 0.00 | 0.00 | 0.00 | 0.00 | 0.05 | 0.05 |
| | 50°C/85%RH 4 weeks later | 0.02 | 0.00 | 0.00 | 0.00 | 0.09 | 0.07 |

According to Table 1, Table 2, and Table 3 wherein the drug concentration was as low as 1.8 %, the uncoated tablets or the film-coated tablets (Examples 1, 2, and 3) which comprise partly pregelatinized starch alone or pregelatinized starch and low substituted hydroxypropylcellulose as a disintegrant produced less of Impurity 1, had good storage stability, and exhibited good moldability. According to Table 4, Table 5, and Table 6 wherein the drug concentration was 7.1 % or 14.3 %, the amount of Impurity 1 produced was lower, compared with the 1.8 % drug concentration formulations mentioned above, but tableting troubles happened to the tablet wherein magnesium stearate was used as a lubricant (Reference example 2). Surprisingly, however, Examples 4, 5, and 6, wherein sodium stearyl fumarate was used as a lubricant and hydroxypropylcellulose was used as a binder, showed no tableting trouble, and showed better storage stability than Reference examples 3 and 4. That is, tablet formulations which comprise hydroxypropylcellulose as a binder, partly pregelatinized starch alone or pregelatinized starch and low substituted hydroxypropylcellulose as a disintegrant, and sodium stearyl fumarate as a lubricant showed good storage stability and moldability even at low drug concentrations.

### Example 7

Uncoated tablet comprising 60 mg of the present compound, wherein hydroxypropylcellulose is used as a binder, and partly pregelatinized starch and low substituted hydroxypropylcellulose are used disintegrants (drug concentration: 21.4 %)

According to the following (11-1), (11-2), (11-3), and (11-4), a granule, a mixed powder, and an uncoated tablet were prepared in order, and a storage stability test (accelerated test) of the uncoated tablet was carried out.

The used amounts shown in parentheses in the following description are each one example for preparing the formulation shown in Example 7. Theoretically, when this preparing method is followed, even if the used amounts are changed at the same ratio and the same procedure is followed, the same preparation can be performed.

### (11-1) Preparation of binding solution (5 % aqueous hydroxypropylcellulose) :

Hydroxypropylcellulose (3.40 g) as a binder was dissolved in purified water (64.64 g) to prepare a binding solution.

### (11-2) Granulation:

The present compound (24.30 g), mannitol (53.95 g), partly pregelatinized starch (14.18 g), and low substituted hydroxypropylcellulose (14.18 g) were thrown in a fluidized bed granulator/dryer machine (FL-LABO/Freund Corporation), and the mixture was granulated by spraying the binding solution prepared in the above (11-1) under the condition defined below to prepare a granule. The obtained granule was mixed with sodium stearyl fumarate to prepare a mixed powder for high drug-concentration (the formula for high drug-concentration is collectively shown in Table 7 below as "Formula C"). Here, the amount of sodium stearyl fumarate to be used was calculated from Formula C based on the amount for preparing the granule shown in Formula C, then which was mixed with the granule.

### Granulation condition

Charge air temperature: 75 - 84°C
Charge air volume: 0.2 m³/min
Spray speed: 4 - 5 g/min
Spray nozzle diameter: 1.0 mm
Spray pressure: 0.12 MPa

### (11-3) Tableting:

The mixed powder prepared in the above (11-2) was tableted with a rotary tablet press VEL2 0308SW2M (KIKUSUI SEISAKUSHO LTD.) to prepare an uncoated tablet.
Punch size: 9 mm
Compression pressure for tableting: 10 kN

### (11-4) Storage stability test:

The uncoated tablet prepared in the above (11-3) was stored under the condition defined in Test 3 shown below.

### Example 8

Uncoated tablet comprising 100 mg of the present compound, wherein hydroxypropylcellulose is used as a binder, and partly pregelatinized starch is used as a disintegrant (drug concentration: 35.7 %)
According to the following (12-1), (12-2), (12-3), and (12-4), a granule, a mixed powder, and an uncoated tablet were prepared in order, and a storage stability test (accelerated test) of the uncoated tablet was carried out.

The used amounts shown in parentheses in the following description are each one example for preparing the formulation shown in Example 8. Theoretically, when this preparing method is followed, even if the used amounts are changed at the same ratio and the same procedure is followed, the same preparation can be performed.

### (12-1) Preparation of binding solution (5 % aqueous hydroxypropylcellulose) :

Hydroxypropylcellulose (3.40 g) as a binder was dissolved in purified water (64.64 g) to prepare a binding solution.

### (12-2) Granulation:

The present compound (40.50 g), mannitol (37.75 g), and partly pregelatinized starch (28.35 g) were thrown in a fluidized bed granulator/dryer machine (FL-LABO/Freund Corporation), and the mixture was granulated by spraying the binding solution prepared in the above (12-1) under the condition defined below to prepare a granule. The obtained granule was mixed with sodium stearyl fumarate to prepare a mixed powder for high drug-concentration (the formula for high drug-concentration is collectively shown in Table 7 below as "Formula C"). Here, the amount of sodium stearyl fumarate to be used was calculated from Formula. C based on the amount for preparing the granule shown in Formula C, then which was mixed with the granule.

### Granulation condition

Charge air temperature: 75°C
Charge air volume: 0.2 m³/min
Spray speed: 4 - 5 g/min
Spray nozzle diameter: 1.0 mm
Spray pressure: 0.12 MPa

### (12-3) Tableting:

The mixed powder prepared in the above (12-2) was tableted with a rotary tablet press VEL2 0308SW2M (KIKUSUI SEISAKUSHO LTD.) to prepare an uncoated tablet.
Punch size: 9 mm
Compression pressure for tableting: 10 kN

### (12-4) Storage stability test:

The uncoated tablet prepared in the above (12-3) was stored under the condition defined in Test 3 shown below.

### Example 9

Uncoated tablet comprising 100 mg of the present compound, wherein hydroxypropylcellulose is used as a binder, and partly pregelatinized starch and croscarmellose sodium are used as disintegrants (drug concentration: 35.7 %)
According to the following (13-1), (13-2), (13-3), and (13-4), a granule, a mixed powder, and an uncoated tablet were prepared in order, and a storage stability test (accelerated test) of the uncoated tablet was carried out.

The used amounts shown in parentheses in the following description are each one example for preparing the formulation shown in Example 9. Theoretically, when this preparing method is followed, even if the used amounts are changed at the same ratio and the same procedure is followed, the same preparation can be performed.

### (13-1) Preparation of binding solution (5 % aqueous hydroxypropylcellulose) :

Hydroxypropylcellulose (3.40 g) as a binder was dissolved in purified water (64.64 g) to prepare a binding solution.

### (13-2) Granulation:

The present compound (40.50 g), mannitol (37.75 g), partly pregelatinized starch (14.18 g), and croscarmellose sodium (14.18 g) were thrown in a fluidized bed granulator/dryer machine (FL-LABO/Freund Corporation), and the mixture was granulated by spraying the binding solution prepared in the above (13-1) under the condition defined below to prepare a granule. The obtained granule was mixed with sodium stearyl fumarate to prepare a mixed powder for high drug-concentration (the formula for high drug-concentration is collectively shown in Table 7 below as "Formula C"). Here, the amount of sodium stearyl fumarate to be used was calculated from Formula C based on the amount for preparing the granule shown in Formula C, then which was mixed with the granule.

### Granulation condition

Charge air temperature: 75 - 80°C
Charge air volume: 0.2 m³/min
Spray speed: 4 - 5 g/min
Spray nozzle diameter: 1.0 mm
Spray pressure: 0.12 MPa

### (13-3) Tableting:

The mixed powder prepared in the above (13-2) was tableted with a rotary tablet press VEL2 0308SW2M (KIKUSUI SEISAKUSHO LTD.) to prepare an uncoated tablet.
Punch size: 9 mm
Compression pressure for tableting: 10 kN

### (13-4) Storage stability test:

The uncoated tablet prepared in the above (13-3) was stored under the condition defined in Test 3 shown below.

### Example 10

Uncoated tablet comprising 100 mg of the present compound, wherein hydroxypropylcellulose is used as a binder, and partly pregelatinized starch and low substituted hydroxypropylcellulose are used as disintegrants (drug concentration: 35.7 %)
According to the following (14-1), (14-2), (14-3), and (14-4), a granule, a mixed powder, and an uncoated tablet were prepared in order, and a storage stability test (accelerated test) of the uncoated tablet was carried out. The used amounts shown in parentheses in the following description are each one example for preparing the formulation shown in Example 10. Theoretically, when this preparing method is followed, even if the used amounts are changed at the same ratio and the same procedure is followed, the same preparation can be performed.

### (14-1) Preparation of binding solution (5 % aqueous hydroxypropylcellulose) :

Hydroxypropylcellulose (3.40 g) as a binder was dissolved in purified water (64.64 g) to prepare a binding solution.

### (14-2) Granulation:

The present compound (40.50 g), mannitol (37.75 g), partly pregelatinized starch (14.18 g), and low substituted hydroxypropylcellulose (14.18 g) were thrown in a fluidized bed granulator/dryer machine (FL-LABO/Freund Corporation), and the mixture was granulated by spraying the binding solution prepared in the above (14-1) under the condition defined below to prepare a granule. The obtained granule was mixed with sodium stearyl fumarate to prepare a mixed powder for high drug-concentration (the formula for high drug-concentration is collectively shown in Table 7 below as "Formula C"). Here, the amount of sodium stearyl fumarate to be used was calculated from Formula C based on the amount for preparing the granule shown in Formula C, then which was mixed with the granule.

### Granulation condition

Charge air temperature: 75°C
Charge air volume: 0.1 - 0.2 m³/min
Spray speed: 4 - 5 g/min
Spray nozzle diameter: 1.0 mm
Spray pressure: 0.12 MPa

### (14-3) Tableting:

The mixed powder prepared in the above (14-2) was tableted with a rotary tablet press VEL2 0308SW2M (KIKUSUI SEISAKUSHO LTD.) to prepare an uncoated tablet.
Punch size: 9 mm
Compression pressure for tableting: 10 kN

### (14-4) Storage stability test:

The uncoated tablet prepared in the above (14-3) was stored under the condition defined in Test 3 shown below.

### Reference example 5

Uncoated tablet comprising 60 mg of the present compound, wherein hydroxypropylcellulose is used as a binder, and partly pregelatinized starch and sodium carboxymethyl starch are used as disintegrants (drug concentration: 21.4 %)

According to the following (15-1), (15-2), (15-3), and (15-4), a granule, a mixed powder and an uncoated tablet were prepared, and a storage stability test (accelerated test) of the uncoated tablet was carried out.

The used amounts shown in parentheses in the following description are each one example for preparing the formulation shown in Reference example 5. Theoretically, when this preparing method is followed, even if the used amounts are changed at the same ratio and the same procedure is followed, the same preparation can be performed.

### (15-1) Preparation of binding solution (5 % aqueous hydroxypropylcellulose) :

Hydroxypropylcellulose (3.40 g) as a binder was dissolved in purified water (64.64 g) to prepare a binding solution.

### (15-2) Granulation:

The present compound (24.30 g), mannitol (53.95 g), partly pregelatinized starch (14.18 g), and sodium carboxymethyl starch (14.18 g) were thrown in a fluidized bed granulator/dryer machine (FL-LABO/Freund Corporation), and the mixture was granulated by spraying the binding solution prepared in the above (15-1) under the condition defined below to prepare a granule. The obtained granule was mixed with sodium stearyl fumarate to prepare a mixed powder for high drug-concentration (the formula for high drug-concentration is collectively shown in Table 7 below as "Formula C"). Here, the amount of sodium stearyl fumarate to be used was calculated from Formula C based on the amount for preparing the granule shown in Formula C, then which was mixed with the granule.

### Granulation condition

Charge air temperature: 75°C
Charge air volume: 0.2 - 0.3 m³/min
Spray speed: 4 - 5 g/min
Spray nozzle diameter: 1.0 mm
Spray pressure: 0.12 MPa

### (15-3) Tableting:

The mixed powder prepared in the above (15-2) was tableted with a rotary tablet press VEL2 0308SW2M (KIKUSUI SEISAKUSHO LTD.) to prepare an uncoated tablet.
Punch size: 9 mm
Compression pressure for tableting: 10 kN

### (15-4) Storage stability test:

The uncoated tablet prepared in the above (15-3) was stored under the condition defined in Test 3 shown below.

### Reference example 6

Uncoated tablet comprising 100 mg of the present compound, wherein hydroxypropylcellulose is used as a binder, and partly pregelatinized starch and sodium carboxymethyl starch are used as disintegrants (drug concentration: 35.7 %)
According to the following (16-1), (16-2), (16-3), and (16-4), a granule, a mixed powder and an uncoated tablet were prepared, and a storage stability test (accelerated test) of the uncoated tablet was carried out.

The used amounts shown in parentheses in the following description are each one example for preparing the formulation shown in Reference example 6. Theoretically, when this preparing method is followed, even if the used amounts are changed at the same ratio and the same procedure is followed, the same preparation can be performed.

### (16-1) Preparation of binding solution (5 % aqueous hydroxypropylcellulose) :

Hydroxypropylcellulose (3.40 g) as a binder was dissolved in purified water (64.64 g) to prepare a binding solution.

### (16-2) Granulation:

The present compound (40.50 g), mannitol (37.75 g), partly pregelatinized starch (14.18 g), and sodium carboxymethyl starch (14.18 g) were thrown in a fluidized bed granulator/dryer machine (FL-LABO/Freund Corporation), and the mixture was granulated by spraying the binding solution prepared in the above (16-1) under the condition defined below to prepare a granule. The obtained granule was mixed with sodium stearyl fumarate to prepare a mixed powder for high drug-concentration (the formula for high drug-concentration is collectively shown in Table 7 below as "Formula C"). Here, the amount of sodium stearyl fumarate to be used was calculated from Formula C based on the amount for preparing the granule shown in Formula C, which was mixed with the granule.

### Granulation condition

Charge air temperature: 75°C
Charge air volume: 0.2 - 0.3 m³/min
Spray speed: 4 - 5 g/min
Spray nozzle diameter: 1.0 mm
Spray pressure: 0.12 MPa

### (16-3) Tableting:

The mixed powder prepared in the above (16-2) was tableted with a rotary tablet press VEL2 0308SW2M (KIKUSUI SEISAKUSHO LTD.) to prepare an uncoated tablet.
Punch size: 9 mm
Compression pressure for tableting: 10 kN

### (16-4) Storage stability test:

The uncoated tablet prepared in the above (16-3) was stored under the condition defined in Test 3 shown below.

### Formula C (Formulae of uncoated tablets in Examples 7, 8, 9, and 10, and Reference examples 5 and 6: drug concentrations 21.4 %, 35.7 %)

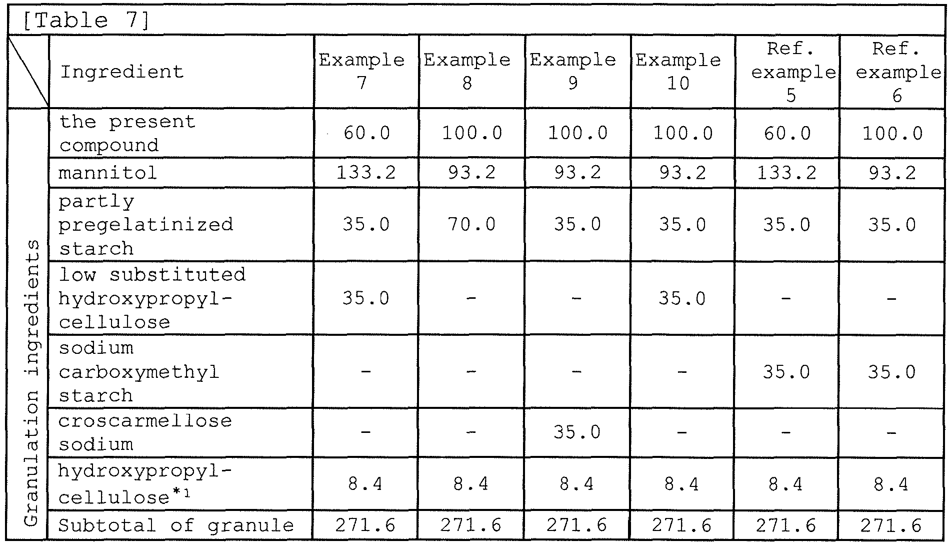

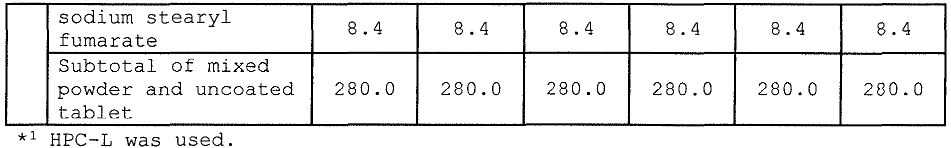

The formulations prepared above were evaluated about their quality in the following manner.

Table 8 shows the evaluation results of the presence or absence of tableting troubles in tableting of Examples 7, 8, 9, and 10 and Reference example 5 and 6. The specific evaluation was made in the manner of <Test 1> mentioned above.

| [Table 8] | | | | | | |
|---|---|---|---|---|---|---|
| | Example 7 | Example 8 | Example 9 | Example 10 | Ref. example 5 | Ref. example 6 |
| Drug concentration (%) | 21.4 | 35.7 | 35.7 | 35.7 | 21.4 | 35.7 |
| Tableting trouble | No | No | No | No | No | No |

| | | | | | | |
|---|---|---|---|---|---|---|
| No: No tableting trouble that affects product appearance | | | | | | |

### <Test 3>

### Storage stability test (Dissolution test)

Each uncoated tablet or film-coated tablet which were prepared above were put in a HDPE Bottle (material: high-density polyethylene (HDPE), internal volume 30 ml, H 61 mm, W 35 mm, L 30 mm), and each bottle in closed state was stored at 40°C/75 % RH in a thermo-hygrostat container for 1, 3, and 6 months. Each sample was tested about dissolution test to evaluate the dissolution rate of the active ingredient. The condition is defined below.

### Test conditions

Detector: UV absorption photometer (wavelength for measurement 290 nm)
Column: in which a stainless tube with an inner diameter of 4.6 mm and a length of about 10 cm is filled with 3 µm octadecylsilylated silica gel for liquid chromatography (YMC-Pack Pro C18 RS).
Column temperature: constant temperature around 40°C
Mobile phase: methanol/0.01 mol/L phosphate buffer (pH 6.8) mixture (4:1)
Flow rate: about 1 mL/min
Time of measuring area: for 70 minutes after injection
Medium for dissolution test: diluted McIlvaine Solution (pH 4.5)
Paddle rotation speed: 50 rpm
Volume of test medium: 900 mL
Sampling: 10, 15, 30, 45, and 60 minutes

The uncoated tablets of Examples 7, 8, 9, and 10, and Reference examples 5 and 6 (initial before storage), and the stored tablets of Examples 7, 8, 9, and 10, and Reference examples 5 and 6 which were stored in the storage conditions defined in Test 3 (samples after acceleration test) were tested about dissolution test, and the results of the dissolution rates (%) are shown in Table 9, Table 10, Table 11, and Table 12.

| [Table 9] | | | | | | | |
|---|---|---|---|---|---|---|---|
| Before storage of accelerate test (Initial) | | Example 7 | Example 8 | Example 9 | Example 10 | Ref. example 5 | Ref. example 6 |
| Drug concentration (%) | | 21.4 | 35.7 | 35.7 | 35.7 | 21.4 | 35.7 |
| Dissolution (%) | 10 min | 94.1 | 76.4 | 64.2 | 80.1 | 75.6 | 47.6 |
| | 15 min | 100.6 | 87.7 | 90.9 | 94.6 | 98.0 | 74.9 |

| [Table 10] | | | | | | | |
|---|---|---|---|---|---|---|---|
| After storage for 1 month in accelerated test | | Example 7 | Example 8 | Example 9 | Example 10 | Ref. example 5 | Ref. example 6 |
| Drug concentration (%) | | 21.4 | 35.7 | 35.7 | 35.7 | 21.4 | 35.7 |
| Dissolution (%) | 10 min | 95.8 | 75.4 | 62.5 | 83.3 | 79.3 | 44.5 |
| | 15 min | 98.9 | 87.8 | 89.1 | 95.4 | 97.9 | 77.6 |

| [Table 11] | | | | | | | |
|---|---|---|---|---|---|---|---|
| After storage for 3 months in accelerated test | | Example 7 | Example 8 | Example 9 | Example 10 | Ref. example 5 | Ref. example 6 |
| Drug concentration (%) | | 21.4 | 35.7 | 35.7 | 35.7 | 21.4 | 35.7 |
| Dissolution (%) | 10 min | 94.4 | 79.2 | 66.8 | 86.1 | 87.4 | 55.0 |
| | 15 min | 97.6 | 90.2 | 90.8 | 95.2 | 97.3 | 78.1 |

| [Table 12] | | | | | | | |
|---|---|---|---|---|---|---|---|
| After storage for 6 months in accelerated test | | Example 7 | Example 8 | Example 9 | Example 10 | Ref. example 5 | Ref. example 6 |
| Drug concentration (%) | | 21.4 | 35.7 | 35.7 | 35.7 | 21.4 | 35.7 |
| Dissolution (%) | 10 min | 98.1 | 82.2 | 73.8 | 88.2 | 93.1 | 62.9 |
| | 15 min | 98.7 | 90.6 | 90.2 | 96.6 | 97.6 | 82.9 |

According to Table 8 about the tablet formulations wherein the drug concentration was 21.4 % or 35.7 %, hydroxypropylcellulose was used as a binder, and sodium stearyl fumarate was used as a lubricant, no tableting troubles affecting the appearance of the product was observed, and the moldability was good. On the other hand, according to Tables 9, 10, 11, and 12, it was observed that when the drug concentration increases from 21.4 % to 35.7 %, the drug dissolution rate tends to slow down. However, when compared between the same drug concentrations (compare Example 7 with Reference example 5, and compare Examples 8, 9, and 10 with Reference example 6), the formulations comprising partly pregelatinized starch alone or pregelatinized starch and low substituted hydroxypropylcellulose or croscarmellose sodium as disintegrants had high dissolution rates after 15 minutes, all of which were good at 85% or more. Further, even after the accelerated test, it did not cause dissolution delay and showed good storage stability.

From the above results, it has become clear that the formulation of the present invention has good dissolution and storage stability, and furthermore, when provided as a tablet, it can suppress tableting troubles from low content to high content of the present compound.

Thus, the present disclosure has exemplified preferred embodiments, but it should be understood that the disclosure is construed in scope solely by the claims.

### INDUSTRIAL APPLICABILITY

The present disclosure can provide an oral solid dosage form comprising (i) N²-{[1-ethyl-6-(4-methylphenoxy)-1H-benzimidazol-2-yl]methyl}-L-alaninamide or a pharmaceutically acceptable salt thereof, or a hydrate or solvate thereof as an active ingredient, (ii) a disintegrant, and (iii) a binder, which can suppress tableting troubles from low content to high content of the present compound, and can also maintain a good dissolution and have an excellent storage stability. Thus, the present invention can stably provide formulations that cover a wide range of doses required in clinical development planning, and further can also produce small tablets whose diameter is, for example, 5.5 mm, thereby can provide an oral solid dosage form that improve the ease of administration and drug compliance of patients and subjects.

## Claims

1. An oral solid dosage form comprising:
(i) N²-{[1-ethyl-6-(4-methylphenoxy)-1H-benzimidazol-2-yl]methyl}-L-alaninamide or a pharmaceutically acceptable salt thereof, or a hydrate or solvate thereof;
(ii) a disintegrant, wherein the disintegrant comprises pregelatinized starches, optionally wherein the pregelatinized starches comprise a pregelatinized starch and/or a partly pregelatinized starch; or optionally wherein the pregelatinized starches comprise a partly pregelatinized starch;
(iii) a binder; and
(iv) a lubricant, wherein the lubricant comprises sodium stearyl fumarate.

2. The oral solid dosage form of claim 1, wherein the disintegrant further comprises a cellulose-type disintegrant; optionally wherein the cellulose-type disintegrant comprises low substituted hydroxypropylcellulose and/or croscarmellose sodium.

3. The oral solid dosage form of claim 1, wherein the disintegrant further comprises low substituted hydroxypropylcellulose.

4. The oral solid dosage form of claim 1, wherein the disintegrant further comprises croscarmellose sodium.

5. The oral solid dosage form of any one of claims 1 to 4, wherein the binder is a water-soluble polymer binder; optionally wherein the water-soluble polymer binder comprises one or a mixture of two or more selected from the group consisting of hydroxypropylcellulose, hydroxypropylmethylcellulose, and polyvinyl alcohol; optionally wherein the water-soluble polymer binder comprises hydroxypropylcellulose.

6. The oral solid dosage form of any one of claims 1 to 5, wherein the content of the disintegrant is 10 - 50 wt % per 100 wt % of the whole dosage form.

7. The oral solid dosage form of any one of claims 2 to 6, wherein the content of the disintegrant other than the pregelatinized starches is 5 - 30 wt % per 100 wt % of the whole dosage form.

8. The oral solid dosage form of any one of claims 1 to 7, wherein the content of the pregelatinized starches is 10 - 30 wt % per 100 wt % of the whole dosage form.

9. The oral solid dosage form of any one of claims 5 to 8, wherein the content of the hydroxypropylcellulose is 1 - 10 wt % per 100 wt % of the whole dosage form.

10. The oral solid dosage form of any one of claims 1 to 9, wherein the content of the N²-{[1-ethyl-6-(4-methylphenoxy)-1H-benzimidazol-2-yl]methyl}-L-alaninamide or a pharmaceutically acceptable salt thereof, or a hydrate or solvate thereof is 0.5 - 70 wt % per 100 wt % of the whole dosage form; optionally wherein the content of the N²-{[1-ethyl-6-(4-methylphenoxy)-1H-benzimidazol-2-yl]methyl}-L-alaninamide or a pharmaceutically acceptable salt thereof, or a hydrate or solvate thereof is 1 - 50 wt % per 100 wt % of the whole dosage form.

11. The oral solid dosage form of any one of claims 1 to 10, further comprising an excipient; optionally wherein the excipient comprises a water-soluble excipient; optionally wherein the water-soluble excipient comprises mannitol.

12. The oral solid dosage form of any one of claims 1 to 11, wherein the content of the sodium stearyl fumarate is 2 - 10 wt % per 100 wt % of the whole dosage form.

13. The oral solid dosage form of claim 11 or claim 12, which is prepared by granulating a mixed powder comprising the N²-{[1-ethyl-6-(4-methylphenoxy)-1H-benzimidazol-2-yl]methyl}-L-alaninamide or a pharmaceutically acceptable salt thereof, or a hydrate or solvate thereof, the disintegrant, and the water-soluble excipient, with a solution of the binder.

14. The oral solid dosage form of any one of claims 1 to 13, which is film-coated with a coating agent.

15. The oral solid dosage form of any one of claims 1 to 14, which is for use in treating and/or preventing pain; optionally wherein the pain is peripheral neuropathic pain.

## Patentansprüche

1. Orale feste Dosierform, umfassend:
(i) N²-{[1-Ethyl-6-(4-methylphenoxy)-1H-benzimidazol-2-yl]methyl}-L-alaninamid oder ein pharmazeutisch annehmbares Salz davon oder ein Hydrat oder Solvat davon;
(ii) ein Auflösungsmittel, wobei das Auflösungsmittel vorgelierte Stärken umfasst, wobei die vorgelierten Stärken gegebenenfalls eine vorgelierte Stärke und/oder eine teilweise vorgelierte Stärke umfassen; oder wobei die vorgelierten Stärken gegebenenfalls eine teilweise vorgelierte Stärke umfassen;
(iii) ein Bindemittel; und
(iv) ein Gleitmittel, wobei das Gleitmittel Natriumstearylfumarat umfasst.

2. Orale feste Dosierform nach Anspruch 1, wobei das Auflösungsmittel weiters ein Auflösungsmittel vom Cellulosetyp umfasst; wobei das Auflösungsmittel vom Cellulosetyp gegebenenfalls niedersubstituierte Hydroxypropylcellulose und/oder Croscarmellosenatrium umfasst.

3. Orale feste Dosierform nach Anspruch 1, wobei das Auflösungsmittel weiters niedersubstituierte Hydroxypropylcellulose umfasst.

4. Orale feste Dosierform nach Anspruch 1, wobei das Auflösungsmittel weiters Croscarmellosenatrium umfasst.

5. Orale feste Dosierform nach einem der Ansprüche 1 bis 4, wobei das Bindemittel ein wasserlösliches Polymerbindemittel ist; wobei das wasserlösliche Polymerbindemittel gegebenenfalls eines oder ein Gemisch aus zwei oder mehr umfasst, die aus der aus Hydroxypropylcellulose, Hydroxypropylmethylcellulose und Polyvinylalkohol bestehenden Gruppe ausgewählt sind; wobei das wasserlösliche Polymerbindemittel gegebenenfalls Hydroxypropylcellulose umfasst.

6. Orale feste Dosierform nach einem der Ansprüche 1 bis 5, wobei der Gehalt des Auflösungsmittels 10 bis 50 Gew.-% bezogen auf 100 Gew.-% der gesamten Dosierform beträgt.

7. Orale feste Dosierform nach einem der Ansprüche 2 bis 6, wobei der Gehalt des Auflösungsmittels, das nicht die vorgelierten Stärken ist, 5 bis 30 Gew.-% bezogen auf 100 Gew.-% der gesamten Dosierform beträgt.

8. Orale feste Dosierform nach einem der Ansprüche 1 bis 7, wobei der Gehalt der vorgelierten Stärken 10 bis 30 Gew.-% bezogen auf 100 Gew.-% der gesamten Dosierform beträgt.

9. Orale feste Dosierform nach einem der Ansprüche 5 bis 8, wobei der Gehalt der Hydroxypropylcellulose 1 bis 10 Gew.-% bezogen auf 100 Gew.-% der gesamten Dosierform beträgt.

10. Orale feste Dosierform nach einem der Ansprüche 1 bis 9, wobei der Gehalt des N²-{[1-Ethyl-6-(4-methylphenoxy)-1H-benzimidazol-2-yl]methyl}-L-alaninamids oder eines pharmazeutisch annehmbaren Salzes davon oder eines Hydrats oder Solvats davon 0,5 bis 70 Gew.-% bezogen auf 100 Gew.-% der gesamten Dosierform beträgt; wobei der Gehalt des N²-{[1-Ethyl-6-(4-methylphenoxy)-1H-benzimidazol-2-yl]methyl}-L-alaninamids oder eines pharmazeutisch annehmbaren Salzes davon oder eines Hydrats oder Solvats davon gegebenenfalls 1 bis 50 Gew.-% bezogen auf 100 Gew.-% der Dosierform beträgt.

11. Orale feste Dosierform nach einem der Ansprüche 1 bis 10, die weiters einen Hilfsstoff umfasst; wobei der Hilfsstoff gegebenenfalls einen wasserlöslichen Hilfsstoff umfasst; wobei der wasserlösliche Hilfsstoff gegebenenfalls Mannit umfasst.

12. Orale feste Dosierform nach einem der Ansprüche 1 bis 11, wobei der Gehalt des Natriumstearylfumarats 2 bis 10 Gew.-% bezogen auf 100 Gew.-% der gesamten Dosierform beträgt.

13. Orale feste Dosierform nach Anspruch 11 oder Anspruch 12, die durch Granulieren eines gemischten Pulvers, umfassend das N²-{[1-Ethyl-6-(4-methylphenoxy)-1H-benzimidazol-2-yl]methyl}-L-alaninamid oder ein pharmazeutisch annehmbares Salz davon oder ein Hydrat oder Solvat davon, das Auflösungsmittel und den wasserlöslichen Hilfsstoff, mit einer Lösung des Bindemittels hergestellt wird.

14. Orale feste Dosierform nach einem der Ansprüche 1 bis 13, die mit einem Beschichtungsmittel filmbeschichtet ist.

15. Orale feste Dosierform nach einem der Ansprüche 1 bis 14, die zur Verwendung bei der Behandlung und/oder Prävention von Schmerzen dient; wobei die Schmerzen gegebenenfalls periphere neuropathische Schmerzen sind.

## Revendications

1. Forme posologique solide orale, comprenant :
(i) du N²-{[1-éthyl-6-(4-méthylphénoxy)-1H-benzimidazol-2-yl]méthyl}-L-alaninamide ou un sel pharmaceutiquement acceptable de celui-ci, ou un hydrate ou solvate de celui-ci ;
(ii) un désintégrant, dans laquelle le désintégrant comprend des amidons prégélatinisés, facultativement dans lequel les amidons prégélatinisés comprennent un amidon prégélatinisé et/ou un amidon partiellement prégélatinisé ; ou facultativement dans lequel les amidons prégélatinisés comprennent un amidon partiellement prégélatinisé ;
(iii) un liant ; et
(iv) un lubrifiant, dans laquelle le lubrifiant comprend du fumarate de stéaryle de sodium.

2. Forme posologique solide orale selon la revendication 1, dans laquelle le désintégrant comprend en outre un désintégrant de type cellulose ; facultativement dans laquelle le désintégrant de type cellulose comprend de l'hydroxypropylcellulose faiblement substituée et/ou de la croscarmellose sodique.

3. Forme posologique solide orale selon la revendication 1, dans laquelle l'agent désintégrant comprend en outre de l'hydroxypropylcellulose faiblement substituée.

4. Forme posologique solide orale selon la revendication 1, dans laquelle le désintégrant comprend en outre de la croscarmellose sodique.

5. Forme posologique solide orale selon l'une quelconque des revendications 1 à 4, dans laquelle le liant est un liant polymère soluble dans l'eau ; facultativement dans laquelle le liant polymère soluble dans l'eau comprend un ou un mélange de deux ou plusieurs choisis dans le groupe comprenant de de l'hydroxypropylcellulose, de l'hydroxypropylméthylcellulose et de l'alcool polyvinylique ; facultativement dans laquelle le liant polymère soluble dans l'eau comprend de l'hydroxypropylcellulose.

6. Forme posologique solide orale selon l'une quelconque des revendications 1 à 5, dans laquelle la teneur du désintégrant est de 10 à 50 % en poids pour 100 % en poids de la forme posologique totale.

7. Forme posologique solide orale selon l'une quelconque des revendications 2 à 6, dans laquelle la teneur du désintégrant autre que les amidons prégélatinisés est de 5 à 30 % en poids pour 100 % en poids de la forme posologique totale.

8. Forme posologique solide orale selon l'une quelconque des revendications 1 à 7, dans laquelle la teneur des amidons prégélatinisés est de 10 à 30 % poids pour 100 % poids de la forme posologique totale.

9. Forme posologique solide orale selon l'une quelconque des revendications 5 à 8, dans laquelle la teneur de l'hydroxypropylcellulose est de 1 à 10 % en poids pour 100 % en poids de la forme posologique totale.

10. Forme posologique solide orale selon l'une quelconque des revendications 1 à 9, dans laquelle la teneur du N2-{[1-éthyl-6-(4-méthylphénoxy)-1H-benzimidazol-2-yl]méthyl}-L-alaninamide ou d'un sel pharmaceutiquement acceptable de celui-ci, ou d'un hydrate ou d'un solvate de celui-ci, est de 0,5 à 70 % en poids pour 100 % en poids de la forme posologique totale ; facultativement dans laquelle la teneur du N2-{[1-éthyl-6-(4-méthylphénoxy)-1H-benzimidazol-2-yl]méthyl}-L-laninamide ou d'un sel pharmaceutiquement acceptable de celui-ci, ou d'un hydrate ou d'un solvate de celui-ci est de 1 à 50 en poids pour 100 % en poids de la forme posologique totale.

11. Forme posologique solide orale selon l'une quelconque des revendications 1 à 10, comprenant en outre un excipient ; facultativement dans laquelle l'excipient comprend un excipient soluble dans l'eau ; facultativement dans laquelle l'excipient soluble dans l'eau comprend du mannitol.

12. Forme posologique solide orale selon l'une quelconque des revendications 1 à 11, dans laquelle la teneur du fumarate de stéaryle de sodium est de 2 à 10 % en poids pour 100 % en poids de la forme posologique totale.

13. Forme posologique solide orale selon la revendication 11 ou la revendication 12, qui est préparée en granulant une poudre mixte comprenant le N²-{[1-éthyl-6-(4-méthylphénoxy)-1H-benzimidazol-2-yl]méthyl}-L-alaninamide ou un sel pharmaceutiquement acceptable de celui-ci, ou un hydrate ou un solvate de celui-ci, le désintégrant et l'excipient soluble dans l'eau, avec une solution du liant.

14. Forme posologique solide orale selon l'une quelconque des revendications 1 à 13, qui est enrobée d'un agent d'enrobage.

15. Forme posologique solide orale selon l'une quelconque des revendications 1 à 14, qui est à utiliser dans le traitement et/ou la prévention de la douleur ; facultativement dans laquelle la douleur est une douleur neuropathique périphérique.
